# EUROPÄISCHE PATENTSCHRIFT

(11) **EP 2 300 397 B1**
(45) Veröffentlichungstag und Bekanntmachung des Hinweises auf die Patenterteilung: **14.05.2014**
(21) Anmeldenummer: 09779508.2
(22) Anmeldetag: 19.05.2009
(51) Int. Cl.: C07C 1/20, C07C 7/04, C07C 11/09

(54) **VERFAHREN ZUR HERSTELLUNG VON ISOBUTEN DURCH SPALTUNG VON MTBE-HALTIGEN GEMISCHEN**
PROCESS FOR PRODUCTION OF ISOBUTENE BY CRACKING MTBE-CONTAINING MIXTURES
PROCÉDÉ DE PRODUCTION D'ISOBUTÈNE PAR SÉPARATION DE MÉLANGES CONTENANT DU MTBE

(30) Priorität: 17.07.2008 DE 102008040511
(43) Veröffentlichungstag der Anmeldung: 30.03.2011
(73) Patentinhaber: Evonik Degussa GmbH, 45128 Essen (DE)
(72) Erfinder: WINTERBERG, Markus, 45711 Datteln (DE); RÖTTGER, Dirk, B-2000 Antwerpen (BE); BUKOHL, Reiner, 45770 Marl (DE); WIEDERHOLD, Holger, 64295 Darmstadt (DE); LUH, Walter, 45770 Marl (DE)
(86) Internationale Anmeldenummer: PCT/EP2009/056069
(87) Internationale Veröffentlichungsnummer: WO 2010/006832

(56) Entgegenhaltungen:
- DE-A1-102006 040 431
- DE-A1-102006 040 434

## Beschreibung

Die vorliegende Erfindung betrifft ein Verfahren zur Herstellung von Isobuten durch Spaltung von MTBE-haltigen Gemischen.

Isobuten ist ein wichtiges Zwischenprodukt für die Herstellung einer Vielzahl organischer Verbindungen, wie beispielsweise für die Herstellung von Butylkautschuk, Polyisobutylen, Isobuten-Oligomeren, verzweigten C₅-Aldehyden, C₅-Carbonsäuren, C₅-Alkoholen und C₅-Olefinen. Weiterhin wird es als Alkylierungsmittel, insbesondere zur Synthese von tert.-Butylaromaten, und als Zwischenprodukt für die Erzeugung von Peroxiden eingesetzt. Darüber hinaus kann Isobuten als Vorstufe für Methacrylsäure und deren Ester verwendet werden.

In üblichen technischen Strömen liegt Isobuten zusammen mit gesättigten und ungesättigten C₄-Kohlenwasserstoffen vor. Aus diesen Gemischen kann Isobuten wegen der geringen Siedepunktsdifferenz bzw. des geringen Trennfaktors zwischen Isobuten und 1-Buten durch Destillation nicht wirtschaftlich abgetrennt werden. Daher wird Isobuten aus technischen Kohlenwasserstoffen häufig dadurch gewonnen, dass Isobuten zu einem Derivat umgesetzt wird, das sich leicht vom übrigen Kohlenwasserstoffgemisch abtrennen lässt, und dass das isolierte Derivat zu Isobuten und Derivatisierungsmittel zurückgespalten wird.

Üblicherweise wird Isobuten aus C₄-Schnitten, beispielsweise der C₄-Fraktion eines Steamcrackers, wie folgt abgetrennt: Nach Entfernung des größten Teils der mehrfach ungesättigten Kohlenwasserstoffe, hauptsächlich des Butadiens, durch Extraktion und Destillation oder Selektivhydrierung zu linearen Butenen wird das verbleibende Gemisch (Raffinat I oder selektiv hydriertes Crack-C₄) mit Alkohol oder Wasser umgesetzt. Bei der Verwendung von Methanol entsteht aus Isobuten Methyl-tert.-butylether (MTBE) und bei Einsatz von Wasser tert.-Butanol (TBA). Nach ihrer Abtrennung können diese Derivate in Umkehrung ihrer Bildung zu Isobuten gespalten werden.

Die Spaltung von Methyl-tert.-butylether (MTBE) zu Isobuten und Methanol kann in Gegenwart saurer oder basischer Katalysatoren in der Flüssigphase bzw. Gas/Flüssig-Mischphase oder in der reinen Gasphase durchgeführt werden.

In US 5 567 860 wird ein Verfahren zur Herstellung von hochreinem Isobuten beschrieben. Hier werden isobutenhaltige C₄-Ströme zunächst mit Methanol verethert, so dass je nach Umsatz ein Gemisch aus MTBE, 2-Methoxybutan (MSBE), nicht umgesetzten C₄-Kohlenwasserstoffen, Methanol, Wasser, Dimethylether (DME), C₄-Oligomeren sowie C₃- und C₅-Kohlenwasserstoffen als Verunreinigung des C₄-Stroms erhalten wird. Dieses Produktgemisch wird destillativ in Leichtsieder, enthaltend C₃-, C₄- und C₅-Kohlenwasserstoffe, Methanol und DME sowie Hochsieder, enthaltend C₄-Oligomere, getrennt. In einem Seitenabzug der Kolonne werden MTBE und 2-Methoxybutan (MSBE) erhalten, welche dann der sauer katalysierten Spaltung zugeführt werden.

In DE 10 2006 040431 wird ein Verfahren für die Herstellung von Isobuten durch MTBE-Spaltung beschrieben. Dabei wird Einsatz-MTBE zusammen mit einem zurückgeführten MTBE-Strom in einer Kolonne durch Abtrennung von Hochsiedern aufgereinigt und das erhaltene MTBE gespalten. Der Austrag der Reaktion wird destillativ in Isobuten, mit anteiligen (azeotropen) Mengen an Methanol, und eine Mischung mit den Hauptbestandteilen Methanol und unumgesetztes MTBE, getrennt. Aus der Methanol/MTBE-Mischung wird anschließend das Methanol größtenteils entfernt und der MTBE-haltige Strom in die Kolonne zur Abtrennung von Hochsiedern zurückgeführt.
Optional werden aus dem Einsatz MTBE Leichtsieder abgetrennt.

Der Offenlegungsschrift DE 10 2006 040430 liegt ein vergleichbares Verfahren zu Grunde. Kennzeichnend sind Aufreinigung des in der Spaltung eingesetzten MTBE auf unter 1000 Massen-ppm MSBE und Konzentrationen an linearen Butenen im erhaltenen Isobuten unter 1000 Massen-ppm. Die Rückführung von nicht umgesetzten MTBE ist optional.

Die Offenlegungsschrift DE 10 2006 040434 beschreibt ein Verfahren zur Spaltung von MTBE, bei dem vom Austrag der Spaltung in einem ersten Schritt Isobuten, mit anteiligen (azeotropen) Mengen an Methanol, abgetrennt wird. Anschließend wird in einer weitern Destillation Methanol als Sumpfprodukt, ein Diisobuten, Methanol und MTBE enthaltender Seitenstrom und eine MTBE und Methanol enthaltender Kopfstrom erhalten, wobei der Kopfstrom in die Spaltung zurückgeführt wird. Die Ausschleusung der im Prozess gebildeten Hochsieder erfolgt hier demnach über einen Seitenstrom.

Die Bildung von Hochsiedern durch Dimerisierung oder Oligomerisierung des Isobutens zu C₄-Oligomeren, sogenannten C₈- und C₁₂-Komponenten, ist eine der bekannten Nebenreaktionen der MTBE-Spaltung. Bei den unerwünschten C₈-Komponenten handelt es sich hauptsächlich um 2,4,4-Trimethyl-1-penten und 2,4,4-Trimethyl-2-penten.

Darüber hinaus setzt sich, besonders an basischen Katalysatoren, ein Teil des bei der Spaltung entstehenden Methanols unter Wasserabspaltung zu DME um.

Die weitere Aufarbeitung der in DE 10 2006 040431, DE 10 2006 040430 und DE 10 2006 040434 erhaltenen methanolhaltigen Isobutenströme sieht daher eine Abtrennung des Methanols durch Extraktion mit Wasser und eine anschließende Destillation vor, bei der DME und Wasser vom Isobuten abgetrennt werden.

Die MTBE-Spaltung in der Gasphase hat den Vorteil, dass sie in der Regel bei höheren Temperaturen abläuft. Das Gleichgewicht der Reaktion von MTBE zu Isobuten und Methanol liegt damit stärker auf Seiten der Produkte, so dass höhere Umsätze erzielt werden können. Wegen der höheren Spalttemperaturen können aber andere und / oder zusätzliche Nebenreaktionen auftreten.

Wie eingangs beschrieben, ist Isobuten ein wichtiges Zwischenprodukt für die Herstellung einer Vielzahl organischer Verbindungen. Die effiziente Herstellung dieser Produkte ist ein Kernbereich der aktuellen industriellen Forschung und stellt dabei gleichzeitig höchste Anforderungen an die Produktreinheit.

Es bestand daher die Aufgabe, ein Verfahren zu entwickeln, bei dem hochreines Isobuten, wie es z. B. für die Herstellung von Polyisobuten (PIB), Butylkautschuk oder auch von Methylmethacrylat (MMA) benötigt wird, zugänglich wird.

Diese Aufgabe wurde gelöst durch ein Verfahren zur Herstellung von Isobuten, aus MTBE-haltigen Gemischen, das folgende Schritte umfasst:
a) Spaltung in einem Reaktor R eines aus einem MTBE-haltigen Einsatzstoff **(1a)** und/oder einem MTBE-haltigen Strom **(13, 5)** erhaltenen Gemisches, liefernd einen Strom **(6)** von Reaktionsprodukten, bestehend aus Isobuten, Methanol, MTBE sowie Nebenprodukten, wobei diese aus
   a1) Hochsiedern, mit einem Siedebereich oberhalb von 55°C bei einem Druck von 0,1 MPa;
   a2) Mittelsiedern, mit einem Siedebereich von 12 bis 55°C bei einem Druck von 0,1 MPa; und
   a3) Leichtsiedern, mit einem Siedebereich unterhalb von 12°C bei einem Druck von 0,1 MPa bestehen;
b) destillative Auftrennung des Stroms **(6)** in einen Strom **(7),** der das Produkt Isobuten und Leichtsieder enthält, und einen Strom **(8),** der MTBE, Methanol, Mittelsieder und Hochsieder enthält;
c) destillative Auftrennung des Stroms **(8)** unter Erhalt eines MTBE-haltigen Stroms **(10, 12)** und eines methanolhaltigen Hochsieder-Stroms **(9**, **11);**
d) Rückführung eines MTBE-haltigen Stroms **(10, 12, 13)** in Schritt a) des Verfahrens, wobei die vollständige oder teilweise Abtrennung der Mittelsieder vor Schritt d) erfolgt aus den mittelsiederreichen Strömen **(10, 12).**

Überraschend wurde nun festgestellt, dass bei der Spaltung von MTBE in der Gasphase Reaktionsgemische entstehen, die Komponenten aufweisen, die bezüglich ihres Normalsiedepunktes zwischen denen der C₄-Kohlenwasserstoffe und MTBE sieden. Als Beispiel können Dimethoxymethan und Isopren genannt werden. Dimethoxymethan kann sich durch Dehydrierung von Methanol zu Formaldehyd und dessen Acetalisierung mit Methanol bilden. Isopren kann aus Formaldehyd und Isobuten gebildet werden. Da es sich, bezogen auf die Siedereihenfolge zu C₄-Kohlenwasserstoffen und MTBE um Komponenten mit mittlerem Siedepunkt handelt, werden diese Komponenten im Folgenden als Mittelsieder bezeichnet. Ihr Siedepunkt liegt typischer Weise bei 0,1 MPa_{(abs)} zwischen 12 °C und 55 °C.

Die Konzentration der bei der Reaktion gebildeten Mittelsieder liegt dabei typischer Weise deutlich unter 500 Massen-ppm bezogen auf den Reaktoraustrag. Nicht gemeint sind mit diesen Mittelsiedern C₄-Kohlenwasserstoffe wie z. B. 1-Buten oder cis- und trans-2-Buten, die z. B. durch Spaltung von MSBE entstehen können und nur knapp oberhalb von Isobuten sieden sowie auch Isobutan, das als Nebenprodukt der Spaltung ebenfalls gebildet werden kann.

**Tabelle 1: Siedepunkte der C₄-Kohlenwasserstoffe bei 0,1 MPa_{(abs)}**

| *C₄-Kohlenwasserstoff* | *Siedepunkt [°C]* |
|---|---|
| Isobutan | -11,74 |
| Isobuten | -7,06 |
| 1-Buten | -6,28 |
| 1,3-Butadien | -4,62 |
| n-Butan | -0,53 |
| trans-2-Buten | 0,87 |
| cis-2-Buten | 3,56 |
| 1,2-Butadien | 10,85 |

Falls die aus den Nebenreaktionen entstehenden Mittelsieder im Verfahren nicht abgetrennt werden, können sich diese im Prozess anreichern und/oder ins Isobutenprodukt gelangen. Für einige Anwendungen des Isobutens, wie z. B. die Herstellung von Polyisobuten (PIB) oder Butylkautschuk, wirken Oxygenate oder konjugierte Diene jedoch störend.

Oxygenate wie Dimethoxymethan weisen ähnliche Eigenschaften auf wie der üblicherweise spezifizierte Dimethylether (DME). Im Gegensatz zu DME siedet Dimethoxymethan bei höheren Temperaturen als Isobuten. Damit können DME und Dimethoxymethan nicht in einem Destillationsschritt von Isobuten abgetrennt werden.
Aus Nebenreaktionen gebildetes Isobutan ist hingegen zumindest in kleineren Konzentrationen in der Regel unkritisch, da es chemisch weitgehend inert ist und übliche Isobutenspezifikationen Konzentrationen bis 1000 ppm im Produkt zulassen (vergleiche Tabelle 5).

In der einschlägigen Literatur ist die Bildung dieser Mittelsieder nicht beschrieben. Daher ist auch kein Verfahren bekannt, dass die Abtrennung dieser Mittelsieder aus dem Reaktionsgemisch vorsieht.

Auch die in US 5,567,860, DE 10 2006 040431, DE 10 2006 040430 und DE 10 2006 040434 beschriebenen Verfahren weisen keinen Schritt für die Abtrennung von Mittelsiedern auf. Nach diesen Verfahren würden diese Komponenten, je nach Fahrweise der nach dem Reaktor angeordneten Kolonne, entweder ganz oder zum Teil ins Kopfprodukt dieser Kolonne und damit in das Produkt Isobuten gelangen.
Bei Abtrennung über das Sumpfprodukt kommt es aber durch die Rückführung des nicht umgesetzten MTBE zu einer Anreicherung der Mittelsieder im Prozess. Passieren die Komponenten den Reaktor dabei ohne weitere Reaktion, würden die Konzentrationen der Mittelsieder bis zu einer Grenzkonzentration ansteigen, bei der sie wieder ins Kopfprodukt der nach dem Reaktor angeordneten Kolonne; und so letztendlich doch in die die Isobutenfraktion gelangen. Sollte es im Reaktor zu Folgereaktionen der Mittelsieder kommen, besteht die Gefahr, dass zusätzliche Komponenten ins Isobuten gelangen oder dass der Katalysator geschädigt wird, beispielsweise durch Verkokung.

Das erfindungsgemäße Verfahren weist im Vergleich zum nächsten Stand der Technik, beschrieben in DE 10 2006 040431 und DE 10 2006 040430, den Vorteil auf, dass im Prozess entstehende Mittelsieder gezielt ausgeschleust werden.

In einer bevorzugten Ausführungsform des Prozesses erfolgt dies zusammen mit den Leicht- und Mittelsiedern des Einsatz MTBE, wodurch der zusätzliche technische und energetische Aufwand gering gehalten wird.

Die Möglichkeit zur Abtrennung der Mittelsieder erweitert zudem den Betrieb der Spaltung auf höhere Temperaturen, da die Bildung der Mittelsieder verstärkt bei hohen Temperaturen stattfindet. Dies kann beispielsweise zur Verlängerung der Katalysatorstandzeit oder zur Verringerung der Katalysatormenge genutzt werden.

### Spaltreaktion

In Schritt a) des erfindungsgemäßen Verfahrens wird MTBE in der Gasphase an einem heterogenen Katalysator zu Isobuten und Methanol gespalten. Dabei können alle festen Katalysatoren eingesetzt werden, die im Temperaturbereich 150 bis 500 °C, insbesondere im Bereich von 200 bis 400 °C, die Spaltung von MTBE zu Isobuten und Methanol bewirken.

Die im erfindungsgemäßen Verfahren eingesetzten Katalysatoren können z. B. Metalloxide, Metallmischoxide, insbesondere solche, die Siliziumoxid und/oder Aluminiumoxid enthalten, Säuren auf Metalloxidträgern oder Metallsalze oder Mischungen davon sein.

Im erfindungsgemäßen Verfahren werden zur Spaltung von MTBE zu Isobuten und Methanol in der Gasphase bevorzugt Katalysatoren verwendet, die formal aus Magnesiumoxid, Aluminiumoxid und Siliziumoxid bestehen. Solche Katalysatoren werden z. B. in US 5 171 920 in Beispiel 4 oder in EP 0 589 557 beschrieben.

Besonders bevorzugt werden Katalysatoren eingesetzt, die formal Magnesiumoxid, Aluminiumoxid und Siliziumdioxid aufweisen, und die einen Anteil an Magnesiumoxid von 0,5 bis 20 Massen-%, bevorzugt von 5 bis 15 Massen-% und besonders bevorzugt von 10 bis 15 Massen-%, einen Anteil an Aluminiumoxid von 4 bis 30 Massen-%, bevorzugt von 10 bis 20 Massen-% und einen Anteil an Siliziumdioxid von 60 bis 95 Massen-%, bevorzugt von 70 bis 90 Massen-% aufweisen. Es kann vorteilhaft sein, wenn der Katalysator neben dem Magnesiumoxid ein Alkalimetalloxid aufweist. Dieses kann z. B. ausgewählt sein aus Na₂O oder K₂O. Vorzugsweise weist der Katalysator als Alkalimetalloxid Na₂O auf. Der bevorzugt eingesetzte Katalysator weist vorzugsweise eine BET-Oberfläche (volumetrisch bestimmt mit Stickstoff gemäß DIN ISO 9277) von 200 bis 450 m²/g, bevorzugt von 200 bis 350 m²/g auf. Wird der Katalysator als aktive Masse auf einem Träger aufgebracht, so weist nur die aktive Masse eine BET-Oberfläche im genannten Bereich auf. Das Material aus Katalysator und Träger kann dagegen je nach Beschaffenheit des Trägers eine deutlich abweichende BET-Oberfläche, insbesondere eine kleinere BET-Oberfläche aufweisen.

Das Porenvolumen des Katalysators beträgt vorzugsweise von 0,5 bis 1,3 ml/g, bevorzugt von 0,65 bis 1,1 ml/g.

Der mittlere Porendurchmesser gemäß DIN 66133 des Katalysators beträgt vorzugsweise von 5 bis 20 nm, bevorzugt von 8 bis 15 nm. Besonders bevorzugt entfällt mindestens 50 %, vorzugsweise über 70 % des Gesamtporenvolumens (Summe des Porenvolumens der Poren mit einem Porendurchmesser von größer gleich 3,5 nm bestimmt mit Quecksilberporosimetrie gemäß DIN 66133) des Katalysators auf Poren mit einem Durchmesser von 3,5 bis 50 nm (Mesoporen).

Im erfindungsgemäßen Verfahren werden bevorzugt Katalysatoren eingesetzt, die eine mittlere Korngröße (bestimmt durch Siebanalyse) von 10 µm bis 10 mm, vorzugsweise 0,5 mm bis 10 mm, besonders bevorzugt eine mittlere Korngröße von 1 bis 5 mm aufweisen. Vorzugsweise werden feste Katalysatoren eingesetzt, die eine mittlere Korngröße d₅₀, von 2 bis 4 mm, insbesondere von 3 bis 4 mm aufweisen.

In dem erfindungsgemäßen Verfahren kann der Katalysator als Formkörper eingesetzt werden. Die Formkörper können jegliche Form einnehmen. Bevorzugt wird der Katalysator als Formköper in Form von Kugeln, Extrudaten oder Tabletten eingesetzt. Die Formkörper weisen vorzugsweise die oben genannten mittleren Korngrößen auf.

Die Herstellung und Verwendung solcher Magnesium-Alumosilikat-Katalysatoren ist in DE 10 2006 040432 beschrieben. Es wird darauf Bezug genommen.

Die Spaltung des MTBE erfolgt in der Gasphase im Temperaturbereich von 150 bis 500 °C, insbesondere 200 bis 400 °C bei Drücken von 0,05 bis 2 MPa, insbesondere bei Drücken von 0,3 bis 1 MPa, ganz besonders bei Drücken von 0,5 bis 0,7 MPa.

Die Spaltung von MTBE in Isobuten und Methanol ist eine endotherme Reaktion. Um eine Partialkondensation von MTBE und Produkten zu vermeiden, ist es zweckmäßig, den Reaktor derart zu betreiben, dass die Minimaltemperatur im Reaktor größer als 150 °C, bevorzugt größer 200 °C ist. Daher liegt die Eingangstemperatur des MTBE, die mittels einem dem Reaktor vorgeschalteten Aufheizer eingestellt werden kann, mindestens bei 150 °C, vorzugsweise mindestens bei 200 °C.

Im Laufe des Betriebes kann es vorteilhaft sein, mit zunehmender Desaktivierung des Katalysators zur Konstanthaltung des Umsatzes die Eingangstemperatur bis auf 500 °C anzuheben. Kann der Umsatz bei Erreichen von 500 °C nicht mehr gehalten werden, so kann es vorteilhaft sein, den Katalysator ganz oder teilweise zu ersetzen.

Der Umsatz des MTBE in Schritt a) des erfindungsgemäßen Verfahrens beträgt zwischen 40 % und 99 %, bevorzugt zwischen 70 % und 98 %, besonders bevorzugt zwischen 85 % und 95 %.

Der Reaktor wird vorzugsweise mit einer Raumgeschwindigkeit (Weight Hourly Space Velocity (WHSV) in Kilogramm Edukt je Kilogramm Katalysator je Stunde) von 0,1 bis 5 h⁻¹, insbesondere von 1 bis 3 h⁻¹ im geraden Durchgang betrieben. Als Reaktoren werden bevorzugt Rohrreaktoren oder Rohrbündelreaktoren, insbesondere solche mit inneren Rohrdurchmessern von 10 bis 60 mm, verwendet. Sie werden vorzugsweise so betrieben, wie in DE 10 2006 040433 beschrieben.

Des Weiteren können Plattenreaktoren für die Durchführung der Spaltungsreaktion eingesetzt werden. Plattenreaktoren sind analog zu Plattenwärmetauschern aufgebaut. Der Abstand der Platten, zwischen denen sich der Katalysator befindet, beträgt dabei bevorzugt 10 - 80 mm.

Der Temperaturabfall in der Katalysatorzone beträgt an jeder beliebigen Stelle in Bezug auf die Eingangstemperatur bevorzugt weniger als 50 °C, besonders bevorzugt weniger als 40 °C und ganz besonders bevorzugt 5 bis 30 °C. Der maximale Temperaturabfall kann durch zahlreiche Parameter, wie z. B. durch die Temperatur des zum Heizen verwendeten Wärmeträgers sowie durch die Geschwindigkeit, mit der der Wärmträger durch den Mantel strömt, eingestellt werden.

Bei der Spaltung von MTBE treten Nebenreaktionen auf. Diese sind entweder auf MTBE oder die Spaltprodukte Isobuten und Methanol zurückzuführen. Standardmäßig tritt bei der MTBE Spaltung die Bildung von Dimethylether (DME) auf. Dabei reagieren zwei Moleküle Methanol zu DME und Wasser. Vorzugsweise wird der erfindungsgemäße Prozess so betrieben, dass die DME-Selektivität der Reaktion zu DME unter 10 % beträgt, bevorzugt unter 4 %. (DME-Selektivität = 2 x [Mol gebildetes DME] / [Mol umgesetztes MTBE]).

Durch Reaktion von Isobuten mit Wasser kann es zudem zur Bildung von tert.-Butanol (TBA) kommen. Bei der Bildung / Spaltung von TBA handelt es sich allerdings um eine Gleichgewichtsreaktion; wird TBA mit dem MTBE in den Reaktor gefahren, so wird dies normalerweise anteilig gespalten.

Eine weitere Nebenreaktion ist die Bildung von C₈-Kohlenwasserstoffen durch Dimerisierung von Isobuten. Diese bestehen hauptsächlich aus Diisobuten, einem Gemisch aus 2,4,4-Trimethylpenten-1 und 2,4,4-Trimethylpenten-2.

Zu den Nebenreaktionen kommen meisten noch parallel ablaufende Reaktionen hinzu, bei denen Verunreinigungen aus dem MTBE reagieren. Darunter fällt beispielsweise die Spaltung von im MTBE enthaltenen 2-Methoxybutan (MSBE). Aus diesem können sich durch Abspaltung von Methanol 1-Buten und 2-Butene bilden. Aus im MTBE enthaltenen 3-Methoxy-1-buten oder 1-Methoxy-2-buten kann in der Spaltung 1,3-Butadien gebildet werden.

### Rohstoffe

Die vorliegende Erfindung ist ein Verfahren zur Herstellung von Isobuten aus MTBE. Es kann MTBE unterschiedlicher Qualität eingesetzt werden. Insbesondere können technisches MTBE verschiedener Qualitäten oder Gemische aus technischem MTBE und Methanol eingesetzt werden.
Um Isobuten hoher Reinheit zu erhalten, ist es vorteilhaft, in Schritt a) des Verfahrens ein Einsatz-MTBE von hoher Reinheit einzusetzen. Dieses kann aus technischem MTBE, welches in der Regel für den Kraftstoffmarkt bestimmt ist, durch Aufreinigung erhalten werden. In bevorzugten Ausführungsformen des erfindungsgemäßen Verfahrens ist diese Aufreinigung Teil des Prozesses, so dass direkt technisches MTBE eingesetzt werden kann. Technisches MTBE (Kraftstoffqualität) ist daher der bevorzugte Einsatzstoff. Tabelle 2 zeigt beispielsweise die typische Zusammensetzung eines technischen MTBE.

**Tabelle 2: Typische Zusammensetzung von technischem MTBE (Kraftstoffqualität).**

| *Komponente* | *Massenanteile [kg*/*kg]* |
|---|---|
| 1-Buten / 2-Butene | 0,001000 |
| Pentane | 0,001500 |
| MTBE | 0,978000 |
| 2-Methoxybutan (MSBE) | 0,003000 |
| Methanol | 0,008500 |
| tert.-Butanol | 0,003000 |
| Wasser | 0,000050 |
| 2,4,4-Trimethylpentene | 0,003300 |

Technisches MTBE kann nach bekannten Verfahren durch Umsetzung von C₄-Kohlenwasserstoffgemischen, aus denen die mehrfach ungesättigten Kohlenwasserstoffe weitgehend entfernt worden sind, beispielsweise Raffinat I oder selektiv hydriertes Crack-C₄, mit Methanol hergestellt werden.

### Aufreinigung der Spaltprodukte

Die Spaltprodukte aus dem Reaktor werden in Schritt b) des erfindungsgemäßen Verfahrens destillativ aufgetrennt. Dabei wird ein Strom 7 erhalten, der mehr als 90 Massen-% Isobuten, Leichtsieder und Anteile Methanol aufweist und in einen weiteren Strom 8, der Methanol, MTBE, Mittelsieder und Hochsieder enthält.

Tabelle 3 zeigt die Reinstoffsiedepunkte von im Reaktoraustrag typischerweise enthaltenen Komponenten bei 0,5 MPa_{(abs)}. Neben Isobuten sind als Leichtsieder weitere C₄-Kohlenwasserstoffe (1-Buten, 2-Butene) und DME enthalten. Isopren und Dimethoxymethan sind bei der Reaktion entstehende Mittelsieder mit Siedepunkten gemäß nachstehender Tabelle. Als Hochsieder, also Komponenten mit einem höheren Siedepunkt als MTBE, sind TBA, Diisobuten und MSBE enthalten.

**Tabelle 3: Reinstoffsiedepunkte von im Reaktoraustrag typischerweise enthaltenen Komponenten bei 0,5 MPa_{(abs)}**

| *Reinstoff* | *Siedetemp.[°C]* | *Typischer Anteil* |
|---|---|---|
| DME | 19,2 | 0,1 - 2 Massen-% |
| Isobuten | 42,7 | 50 - 60 Massen-% |
| 1-Buten | 43,4 | 0 - 150 Massen-ppm |
| trans-2-Buten | 51,4 | 0 - 750 Massen-ppm (Summe 2-Butene) |
| cis-2-Buten | 54,1 | |
| Isopren | 90,4 | 10 - 300 Massen-ppm |
| Dimethoxymethan | 95,9 | 5 - 150 Massen-ppm |
| Methanol | 111,5 | 30 - 37 Massen% |
| MTBE | 113,8 | 3 - 25 Massen% |
| MSBE | 120,8 | 0 - 1700 Massen-ppm |
| TBA | 131,3 | 0 - 1000 Massen-ppm |
| Diisobuten | 171,2 | 100 - 1000 ppm |

Durch entsprechende Auslegung und Betriebsweise der destillativen Trennung in Schritt b) des erfindungsgemäßen Verfahrens ist es möglich, die Mittelsieder zum größten Teil oder vollständig im Strom **8** anzureichern, so dass der Isobutenreiche Strom 7 frei bzw. nahezu frei von diesen Mittelsiedern ist.

Die destillative Auftrennung der Spaltprodukte **6** erfolgt dabei bevorzugt in genau einer Destillationskolonne. Diese Destillationskolonne weist vorzugsweise von 20 bis 55 theoretische Trennstufen, bevorzugt von 25 bis 45 und besonders bevorzugt von 30 bis 40 theoretische Trennstufen auf. Das Rücklaufverhältnis beträgt, in Abhängigkeit von der realisierten Stufenzahl, der Zusammensetzung des Reaktoraustrags und der erforderlichen Reinheiten von Destillat und Sumpfprodukt, vorzugsweise kleiner 5, bevorzugt kleiner 1. Der Betriebsdruck der Kolonne kann vorzugsweise zwischen 0,1 und 2,0 MPa_{(abs)} eingestellt werden. Dabei ist es vorteilhaft, die Kolonne bei einem niedrigeren Druck als den Druck, mit dem der Spaltungsreaktor (R) betrieben wird, zu betreiben. Dann können die Reaktionsprodukte **6** gasförmig oder teilweise gasförmig, nach partieller Kondensation, in die Destillationskolonne überführt werden. Auf einen Verdichter zur Anhebung des Drucks oder eine vollständige Kondensation kann so verzichtet werden.

Besonders bevorzugt werden die Reaktionsprodukte **6** nach partieller Kondensation in die Destillationskolonne überführt. Dabei werden bevorzugt 30-70 %, besonders bevorzugt 40 - 70 % des Gasstroms kondensiert. Der nicht kondensierte Gasstrom wird direkt, der kondensierte falls notwendig nach Druckerhöhung durch eine Pumpe, in die Kolonne eingebracht.
Die Einspeisung der Gasphase und der Flüssigphase kann dabei an gleicher oder unterschiedlicher Stelle der Kolonne erfolgen. Bevorzugt erfolgt die Einspeisung der Flüssigphase auf dem gleichen Boden oder ein bis fünf Böden unterhalb der Einspeisung der Gasphase.
Die bei der partiellen Kondensation der Gasphase freiwerdende Energie wird bevorzugt an anderer Stelle des Prozesses genutzt, beispielsweise zur Beheizung einer Kolonne oder zur Vorwärmung des Reaktorzulaufs.

Um Isobuten am Kolonnenkopf gegen Kühlwasser kondensieren zu können, ist ein Druck von ca. 0,5 MPa_{(abs)} notwendig. Wird die Spaltung beispielsweise bei einem Druck von 0,65 MPa_{(abs)} betrieben, kann es vorteilhaft sein, wenn die Destillationskolonne mit einem Betriebsdruck von 0,55 bis 0,6 MPa_{(abs)} durchgeführt wird. Zur Beheizung des Verdampfers der Kolonne kann z. B. 0,4 MPa-Dampf eingesetzt werden. Als Sumpfprodukt wird der Strom **8** erhalten, als Kopfprodukt der Strom **7.** Dieser enthält mehr als 90 Massen-%, vorzugsweise mehr als 95 Massen-% Isobuten bezogen auf das gesamte Kopfprodukt.

Optional kann die Kolonne als Reaktivdestillation ausgeführt werden. Dies hat den Vorteil, dass der MTBE-Umsatz im gesamten Verfahren dadurch erhöht werden kann, dass ein Teil des im Spaltreaktor nicht umgesetzten MTBEs im Reaktionsteil der Reaktivdestillationskolonne zu Isobuten und Methanol gespalten wird. Die Ausführung der Kolonne als Reaktivdestillation ist unter anderem in der Offenlegungsschrift DE 102006040430 beschrieben.

Der Gehalt an linearen Butenen im Strom **7** beträgt bezogen auf die C₄-Olefinfraktion vorzugsweise weniger als 10000 Massen-ppm, bevorzugt weniger als 5000 Massen-ppm und besonders bevorzugt weniger als 1000 Massen-ppm. Dabei beträgt der Gehalt an 1-Buten in Strom **7** bezogen auf die C₄-Olefinfraktion vorzugsweise weniger als 5000 Massen-ppm, bevorzugt weniger als 1000 Massen-ppm und besonders bevorzugt weniger als 500 Massen-ppm. Der Gehalt an 2-Butenen (Summe der beiden 2-Butene) beträgt bezogen auf die C₄-Olefinfraktion vorzugsweise ebenfalls weniger als 5000 Massen-ppm, bevorzugt weniger als 2000 Massen-ppm und besonders bevorzugt weniger als 500 Massen-ppm.

Eine, nicht erfindungsgemäße, Abtrennung der Mittelsieder zusammen mit dem Isobuten und den Leichtsiedern ist technisch ebenfalls möglich. Aus dem Isobuten können dann die Mittelsieder, gegebenenfalls nach Abtrennung von Methanol, destillativ abgetrennt werden. Dabei fallen diese allerdings als Sumpfstrom an, wohingegen die gesamte Isobutenmenge als Kopfprodukt erhalten wird. Technisch ist dies weniger vorteilhaft.

Vom im Schritt b) des Verfahrens erhaltenen Strom **8** wird in Schritt c) der größte Teil des enthaltenen Methanols abgetrennt. Nachfolgende Tabelle 4 zeigt die Siedepunkte der Reinstoffe MTBE, MSBE, Methanol, TBA und Diisobuten sowie exemplarisch für bei der Reaktion entstandene Mittelsieder die Siedepunkte von Isopren und Dimethoxymethan bei 0,1 MPa_{(abs)}. Für das in zwei Isomeren vorkommende Diisobuten wurde exemplarisch der Siedepunkt von 2,4,4-Trimethylpent-1-en aufgeführt. Zu erkennen ist, dass in der Reihenfolge Isopren, Dimethoxymethan, MTBE, MSBE, Methanol, TBA und Diisobuten der Siedepunkt steigt. Gleichzeitig bilden aber die Komponenten Isopren, MTBE, MSBE und Diisobuten mit Methanol Minimum-Azeotrope. Die Siedepunkte dieser Azeotrope und die Zusammensetzung ist ebenfalls in Tabelle 4 aufgeführt, wobei die Azeotrope mit der Property-Methode "UNIFAC-DMD" (s. J. Gmehling, J. Li, and M. Schiller, Ind. Eng. Chem. Res. 32, (1993), pp. 178-193) mit dem stationären Simulationsprogramm ASPEN Plus (Version 12.1 der Firma AspenTech) berechnet wurden. Unter dieser Randbedingung ist das Isopren-Methanol-Azeotrop der Leichtsieder im System und kann bei Durchführung der Trennung in einer einzigen Kolonne als Kopfprodukt gewonnen werden. Will man aber nahezu reines Methanol als Sumpfprodukt generieren, muss man zusätzlich die Reinstoffe Isopren und Dimethoxymethan und die Azeotrope MTBE-Methanol, MSBE-Methanol und Diisobuten-Methanol über Kopf destillieren. Als einzige Nebenkomponente verbleibt - neben dem in der Tabelle nicht aufgeführten Wasser und dem Wertprodukt Methanol - TBA im Sumpfprodukt.

**Tabelle 4: Siedepunkte der Reinstoffe und der Azeotrope mit Methanol bei 0,1 MPa_{(abs)}; die Azeotrope wurden mit der Property-Methode "UNIFAC-DMD" berechnet.**

| *Reinstoff* / *Azeotrop* | *Siedetemp. [°C]* | *Zusammensetzung [Massen-%]* | |
|---|---|---|---|
| Azeotrop Isopren + Methanol | 30,5 | Isopren / Methanol | 91,69 / 8,31 |
| Reinstoff Isopren | 33,7 | | |
| Reinstoffe Dimethoxymethan | 41,6 | | |
| Azeotrop MTBE + Methanol | 50,5 | MTBE / Methanol: | 86,22 / 13,78 |
| Azeotrop MSBE + Methanol | 54,1 | 2-Methoxybutan / Methanol: | 80,40 / 19,60 |
| Reinstoff MTBE | 54,7 | - | |
| Azeotrop Methanol + Diisobuten | 59,2 | Methanol / Diisobuten | 47,84 / 52,16 |
| Reinstoff MSBE | 60,8 | - | |
| Reinstoff Methanol | 64,2 | - | |
| Reinstoff TBA | 82,1 | - | |
| Reinstoff 2,4,4-Trimethylpenten-1 | 101,1 | - | |

Die destillative Abtrennung des Methanols aus dem Sumpfprodukt **8** erfolgt in einer oder mehreren Destillationskolonne(n), vorzugsweise in einer Destillationskolonne.
Bei Verwendung einer Destillationskolonne weist diese vorzugsweise 20 bis 75 theoretische Trennstufen, bevorzugt 30 bis 65 und besonders bevorzugt 35 bis 50 auf. Es kann vorteilhaft sein, wenn die Kolonne in Abhängigkeit von der realisierten Stufenzahl und des im Spaltungsreaktor erzielten MTBE-Umsatzes, mit einem Rücklaufverhältnis, von kleiner 10, bevorzugt von 0,5 bis 5 betrieben wird. Der Betriebsdruck der Kolonne wird vorzugsweise auf einen Wert im Bereich von 0,05 bis 1 MPa_{(abs)}, bevorzugt von 0,1 bis 0,3 MPa_{(abs)} eingestellt. Zur Beheizung der Kolonne kann z. B. 0,4 MPa Dampf eingesetzt werden. Die Kondensation kann, je nach gewähltem Betriebsdruck, gegen Kühlsole, Kühlwasser oder Luft erfolgen.

Das in Schritt c) des Verfahrens abgetrennte Methanol enthält vorzugsweise größer 97, besonders bevorzugt größer 99 Massen-% Methanol. Der TBA-Gehalt im Sumpfprodukt beträgt vorzugsweise zwischen 500 und 2000 Massen-ppm und der Wassergehalt vorzugsweise von 0,5 bis 0,8 Massen-%. Die im Reaktionsteil entstandenen Mittelsieder, z. B. Isopren und Dimethoxymethan, sind vorzugsweise mit kleiner 200 ppm, bevorzugt kleiner 100 ppm und besonders bevorzugt mit kleiner 50 ppm enthalten. Somit hat das Methanol eine so hohe Reinheit, dass es für übliche technische Synthesen, beispielsweise Veresterungen oder Veretherungen, eingesetzt werden kann. Bei Bedarf kann das Methanol aber auch in einem weiteren Destillationsschritt auf noch höhere Reinheiten aufkonzentriert werden.

Der in Schritt c) des Verfahrens nach der Abtrennung von Methanol erhaltene Strom 10 enthält neben dem Hauptbestandteil MTBE noch Methanol und die im Reaktionsteil entstandenen Mittelsieder, beispielsweise Isopren und/oder Dimethoxymethan. Erfindungsgemäß werden die Mittelsieder von Strom **10** abgetrennt, bevor er in Schritt a) des Verfahrens zurückgeführt wird.
Die destillative Abtrennung der Mittelsieder aus Strom **10** kann in einer oder in mehreren Kolonnen erfolgen. Dabei kann es zudem vorteilhaft sein, die Abtrennung der Mittelsieder aus Strom **10** mit der Aufreinigung des Einsatz-MTBE zu kombinieren.

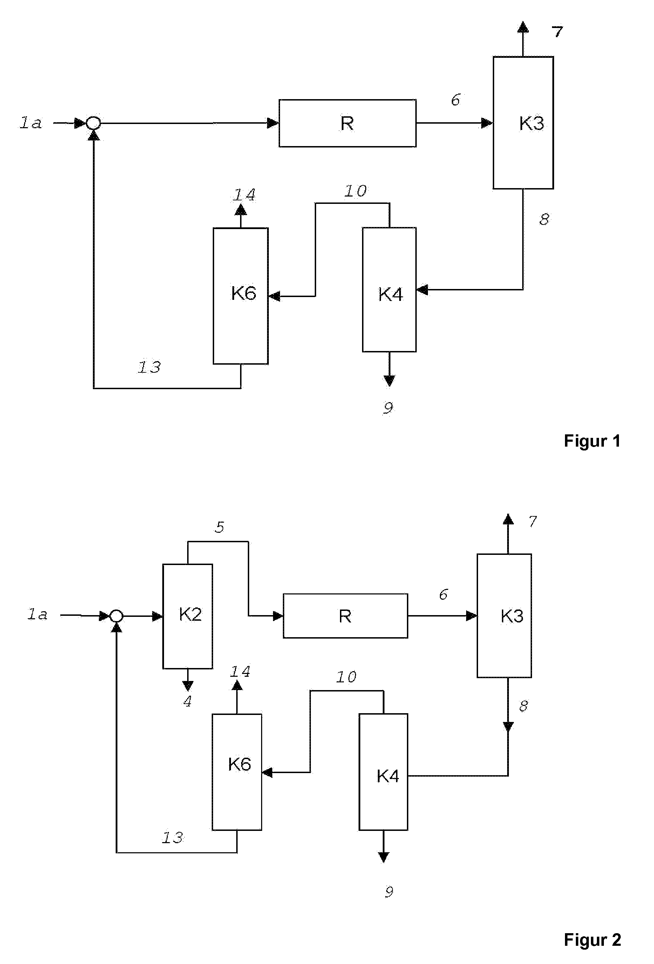

Anhand der Figuren 1 bis 4 wird die vorliegende Erfindung näher erläutert, ohne dass die Erfindung auf die dort beispielhaft abgebildeten Ausführungsarten beschränkt sein soll.

Ein Blockschema einer bevorzugten Ausführungsform, mit der das erfindungsgemäße Verfahren durchgeführt werden kann, ist in Figur 1 dargestellt.
Das Einsatz MTBE **1a** wird zusammen mit dem Rückstrom **13** in den Spaltungsreaktor (R) geführt. Das Spaltungsprodukt **6** wird in der Kolonne (K3) in ein Kopfprodukt **7,** dass das gebildete Isobuten, DME und aufgrund der Azeotropbildung zwischen Isobuten und Methanol Anteile an Methanol enthält, und in ein Sumpfprodukt **8** mit dem nicht umgesetzten MTBE und den größeren Teil des entstandenen Methanols getrennt. Das Sumpfprodukt **8** enthält auch die im Reaktionsteil entstandenen Mittelsieder, beispielsweise Isopren und/oder Dimethoxymethan.
Aus dem Strom **8** wird in der Kolonne (K4) der größte Teil des Methanols und Wasser als Sumpfprodukt **9** abgetrennt. In Kolonne (K6) werden aus dem Strom **10** die Mittelsieder **14** abgetrennt und der hauptsächlich MTBE-haltige Rückstrom **13** wird in die Spaltung zurückgeführt.

Eine weitere bevorzugte Ausführungsform des Verfahrens ist in Figur 2 dargestellt. Hierbei werden vor der Zuführung in den Spaltungsreaktor (R) aus dem Einsatz-MTBE **1a** und dem MTBE-haltigen Rückstrom **13** in der Kolonne (K2) zusätzlich Hochsieder **4** zumindest teilweise abgetrennt. Als Destillat wird der Strom **5** erhalten, der in den Spaltungsreaktor (R) geführt wird. Die weitere Aufarbeitung erfolgt wie zuvor unter Figur 1 beschrieben.

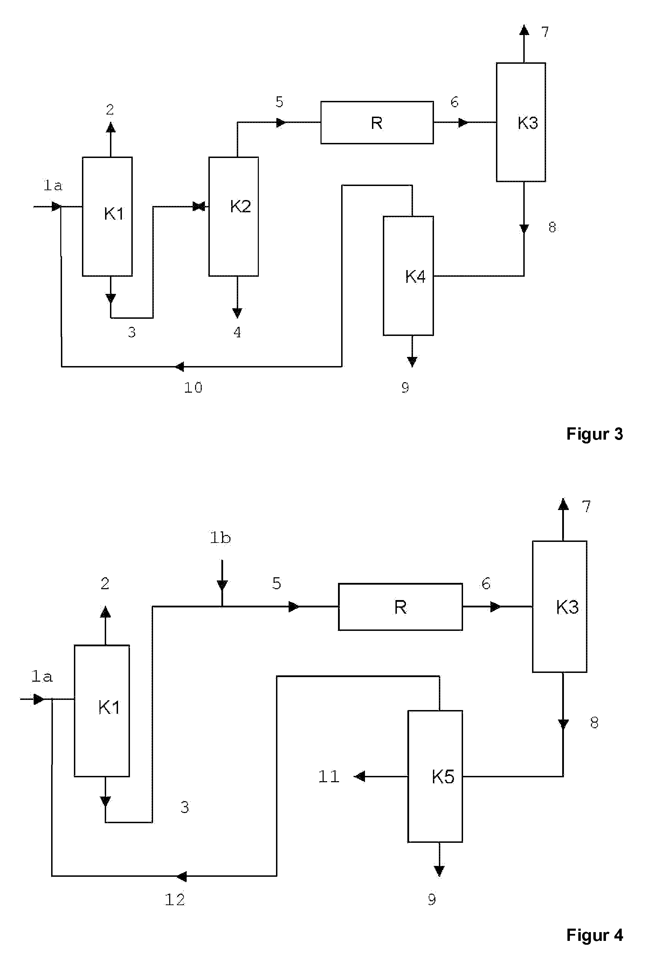

In Figur 3 ist eine weitere bevorzugte Ausführungsform des Verfahrens wiedergegeben. Das Einsatz-MTBE **1a** wird zusammen mit dem Strom **10** in der Kolonne (K1) fraktioniert. Der Rückstrom **10** enthält neben dem nicht umgesetzten MTBE und Methanol auch die im Reaktionsteil (R) entstandenen Mittelsieder, z. B. Isopren und/oder Dimethoxymethan. In der Kolonne (K1) werden diese Mittelsieder zusammen mit den gegebenenfalls im Einsatz-MTBE **1a** enthaltenen Leicht- und/oder Mittelsiedern als Kopfprodukt **2** anteilig abgetrennt. Das Sumpfprodukt **3** wird in die Kolonne (K2) geleitet. Dort werden enthaltene Hochsieder (Diisobuten, MSBE) zumindest teilweise als Sumpfprodukt **4** abgetrennt. Das Kopfprodukt 5 wird in den Spaltungsreaktor (R) geleitet. Das Spaltungsprodukt 6 wird in der Kolonne (K3) in ein Kopfprodukt **7,** dass das gebildete Isobuten, DME und aufgrund der Azeotropbildung zwischen Isobuten und Methanol Anteile an Methanol enthält, und in ein Sumpfprodukt **8** mit dem nicht umgesetzten MTBE und den größeren Teil des entstandenen Methanols getrennt. Das Sumpfprodukt **8** enthält auch die im Reaktionsteil entstandenen Mittelsieder, z. B. Isopren und/oder Dimethoxymethan.
Aus dem Strom **8** wird in der Kolonne (K4) der größte Teil des Methanols und Wasser als Sumpfprodukt **9** abgetrennt. Das Kopfprodukt **10,** das MTBE, ein Anteil des Methanols und die im Reaktionsteil entstandenen Mittelsieder, beispielsweise Isopren und/oder Dimethoxymethan, enthält, wird zusammen mit dem Einsatz-MTBE **1a** in der Kolonne (K1) aufgereinigt.

Optional können die Kolonnen (K1) und (K2) durch eine einzige Trennwandkolonne ersetzt sein.
Die Ausführung des Verfahrens nach Figur 3 bietet den Vorteil, dass technisches MTBE direkt in das Verfahren eingesetzt werden kann. Enthaltene Leichtsieder werden in der Kolonne (K1) zusammen mit den im Prozess entstandenen Mittelsiedern abgetrennt. Durch diese Kombination ergibt sich der potentielle Vorteil, eine Kolonne einzusparen. Die Hochsieder aus dem Prozess und aus dem Einsatz MTBE (beispielsweise MSBE) werden gemeinsam in Kolonne (K2) abgetrennt.

Ein Blockschema einer weiteren bevorzugten Ausführungsform einer Anlage, in der das erfindungsgemäße Verfahren durchgeführt werden kann, ist in Figur 4 dargestellt. Das Einsatz-MTBE **1a** wird zusammen mit dem MTBE enthaltenden Rückstrom **12** in der Kolonne (K1) fraktioniert. Dieser Rückstrom **12** enthält neben dem nicht umgesetzten MTBE und Methanol auch die im Reaktionsteil (R) entstandenen Mittelsieder, z. B. Isopren und/oder Dimethoxymethan. In der Kolonne (K1) werden diese Mittelsieder zusammen mit den gegebenenfalls im Einsatz-MTBE enthaltenen Leichtsiedern (C₄- und C₅-Kohlenwasserstoffe) als Kopfprodukt **2** anteilig abgetrennt. Das Sumpfprodukt wird in den Spaltungsreaktor (R) geleitet. Das Spaltungsprodukt **6** wird in der Kolonne (K3) in ein Kopfprodukt **7,** dass das gebildete Isobuten, DME und aufgrund der Azeotropbildung zwischen Isobuten und Methanol Anteile an Methanol enthält, und in ein Sumpfprodukt **8** mit dem nicht umgesetzten MTBE und den größeren Teil des entstandenen Methanols getrennt. Das Sumpfprodukt **8** enthält auch die im Reaktionsteil entstandenen Mittelsieder, z. B. Isopren und/oder Dimethoxymethan.
Die Kolonne (K5) stellt eine mögliche Ausführung des Schrittes c) des erfindungsgemäßen Verfahrens da. In ihr wird Strom **8** in ein Sumpfprodukt **9,** in einen Seitenstrom **11** und in ein Kopfprodukt **12** getrennt. Das Sumpfprodukt **9** enthält vorzugsweise über 98 % Methanol. Mit dem Seitenstrom **11** werden Diisobuten und gegebenenfalls weitere Hochsieder, beispielsweise MSBE, ausgeschleust. Das MTBE, ein Anteil des Methanols und die im Spaltungsreaktor entstandenen Mittelsieder, z. B. Isopren und/oder Dimethoxymethan, werden als Kopfprodukt **12** erhalten und zusammen mit dem Einsatz-MTBE **1a** in die Kolonne (K1) eingespeist.
Die Ausführungsform nach Figur 4 ist vor allem für solche MTBE-Einsatzgemische interessant, die geringe Mengen an Diisobuten und MSBE enthalten. In diesem Fall kann auf die Kolonne K2 zur Abtrennung dieser Komponenten vor dem Reaktionsteil verzichtet werden, womit sich das Verfahren vereinfacht. Enthält das Einsatz-MTBE keine Leichtsieder, kann es als Strom **1b** direkt in den Spaltreaktor geleitet werden. In der Kolonne K1 werden dann nur die Mittelsieder als Strom **2** abgetrennt.

Erfindungsgemäß ist allen Ausführungsformen gemeinsam, dass es eine Kolonne gibt, in der im Spaltungsreaktor (R) entstandene Mittelsieder über Kopf abgetrennt werden.

Die destillative Abtrennung von den im Spaltungsreaktor (R) entstandenen Mittelsiedern, z. B. Isopren und/oder Dimethoxymethan, kann anstatt in den Kolonnen (K6) oder (K1) auch aus einem anderen Strom erfolgen, beispielsweise aus Strom **8** (Figur 1) oder Strom **5** (Figur 2). Auch diese Optionen sind Ausführungsformen des erfindungsgemäßen Verfahrens, auch wenn sie nicht separat betrachtet werden.

### Stofftrennung

Im Folgenden werden für die in den Figuren 1 bis 4 bezeichneten Kolonnen K1, K2, K5 und K6 bevorzugte Ausführungsformen und Betriebsparameter angegeben.

### Kolonne (K1), Figuren 3 & 4

In der Kolonne (K1) werden erfindungsgemäß aus dem überwiegend aus MTBE bestehenden Rückstrom **(10** oder **12)** die im Reaktionsteil entstandenen Mittelsieder, z. B. Dimethoxymethan und/oder Isopren, abgetrennt. Zusätzlich können Leichtsieder (C₄- und C₅-Kohlenwasserstoffe) aus dem technischen MTBE entfernt werden.

Vorzugsweise wird die Stofftrennung in einer Destillationskolonne durchgeführt, die 30 bis 85 theoretische Trennstufen, bevorzugt 40 bis 75 und besonders bevorzugt 40 bis 60 theoretische Trennstufen aufweist. Vorzugsweise wird die Kolonne, in Abhängigkeit von der realisierten Stufenzahl, der Zusammensetzung des eingesetzten MTBE und der erforderlichen Reinheit an C₄- und C₅-Kohlenwasserstoffen und der im Reaktionsteil entstandenen Mittelsieder, z. B. Dimethoxymethan und/oder Isopren, mit einem Rücklaufverhältnis zwischen 50 und 350, insbesondere zwischen 120 und 300 betrieben. Die Kolonne wird vorzugsweise mit einem Betriebsdruck von 0,2 bis 0,6 MPa_{(abs)}, bevorzugt von 0,3 bis 0,4 MPa_{(abs)} betrieben. Zur Beheizung der Kolonne kann z. B. Dampf eingesetzt werden. Die Kondensation kann, je nach gewähltem Betriebsdruck, gegen Kühlsole, Kühlwasser oder Luft erfolgen. Der Kopfbrüden der Kolonne kann vollständig oder nur teilweise kondensiert werden, so dass das Kopfprodukt **2** entweder flüssig oder dampfförmig abgezogen werden kann. Das Kopfprodukt **2** kann thermisch verwertet werden oder als Einsatzstoff einer Synthesegasanlage genutzt werden.

### Kolonne (K2), Figuren 2 & 3

In der Kolonne (K2) wird der MTBE aufweisende Strom aufdestilliert, wobei Schwersieder, insbesondere Diisobuten und/oder MSBE, vom MTBE abgetrennt werden. Wenn in der Kolonne primär nur Diisobuten abgetrennt werden soll, kann es vorteilhaft sein, wenn die Kolonne 15 bis 60 theoretische Trennstufen, vorzugsweise 20 bis 55 und bevorzugt 30 bis 45 theoretische Trennstufen aufweist. Das Rücklaufverhältnis, im Rahmen der vorliegenden Erfindung definiert als der Massenstrom des Rücklaufs geteilt durch den Massenstrom des Destillats, wird, in Abhängigkeit von der realisierten Stufenzahl, der Zusammensetzung des eingesetzten MTBE und der erforderlichen Reinheit, vorzugsweise auf einen Wert von 0,5 bis 7, bevorzugt von 1 bis 4 eingestellt.

Soll in der Kolonne Diisobuten und zusätzlich MSBE abgetrennt werden, weist die eingesetzte Destillationskolonne vorzugsweise von 50 bis 140 theoretische Trennstufen, bevorzugt von 60 bis 120 und ganz besonders bevorzugt von 80 bis 110 auf. Das Rücklaufverhältnis beträgt, in Abhängigkeit von der realisierten Stufenzahl, der Zusammensetzung des eingesetzten MTBE und der erforderlichen Reinheit, vorzugsweise von 1 bis 20, bevorzugt von 3 bis 10.

Zur Erhöhung der Flexibilität im Hinblick auf die Qualität des Einsatz-MTBE **1a** und auf die geforderte Reinheit des hergestellten Isobutens kann es besonders vorteilhaft sein, eine Kolonne vorzusehen, mit der beide Stoffe abgetrennt werden können, also eine Kolonne, die vorzugsweise 50 bis 140 theoretische Trennstufen aufweist. Selbst wenn die Abtrennung von MSBE nicht notwendig sein sollte, muss die größere Kolonne kein Nachteil sein, da ein Teil des höheren Kapitaleinsatzes für die größere Kolonne durch Energieeinsparung (Verringerung des Rücklaufverhältnisses) kompensiert werden kann.

Der Betriebsdruck kann, unabhängig davon, ob nur Diisobuten oder zusätzlich MSBE abgetrennt werden soll, vorzugsweise von 0,1 bis 2,0 MPa_{(abs)} betragen. Wenn die Spaltung des Kopfproduktes **5** im Spaltreaktor in der Gasphase bei erhöhtem Druck erfolgt, kann es vorteilhaft sein, die Destillation bei höherem Druck durchzuführen, wobei in diesem Fall der Kopfkondensator vorzugsweise als Partial kondensator betrieben wird und das Kopfprodukt **5** dampfförmig abgezogen wird. Beträgt der Reaktionsdruck im Spaltreaktor beispielsweise 0,7 MPa_{(abs)}, so sollte der Destillationsdruck vorzugsweise mindestens 0,75 MPa_{(abs)} betragen. Bei Betriebsdrucken von größer 1,3 MPa_{(abs)} kann mit der Kondensationswärme Niederdruck- (ND-) Dampf erzeugt werden, mit dem andere Kolonnen des Verfahrens beheizt werden können. Zur Beheizung der Kolonne kann, je nach gewähltem Betriebsdruck, Dampf oder Wärmeträgeröl eingesetzt werden. Das Sumpfprodukt **4** kann neben den Schwersiedern, wie z. B. MSBE und Diisobuten, auch MTBE enthalten. Dieses Gemisch kann thermisch verwertet werden, als Einsatzstoff für eine Synthesegasanlage genutzt werden oder direkt oder nach Hydrierung als Treibstoffkomponente verwendet werden.

Die Zusammensetzung des Zulaufs zur Kolonne (K2) kann abhängig vom Einsatz-MTBE **1a** und des Umsetzungsgrads des MTBE im Spaltreaktor (R) (gerader Durchgang) variieren. Bei Einsatz eines MTBE der Zusammensetzung nach Tabelle 2 und einem MTBE-Umsatz, der zwischen 50 und 95 % liegt, weist der Zulauf zur Kolonne zwischen 85 und 97 Massen-% MTBE auf, der Methanolgehhalt liegt zwischen 2 und 12 Massen-%. Der Diisobutengehalt liegt zwischen 1500 und 5000 Massen-ppm, der Gehalt an MSBE liegt zwischen 3500 und 5000 Massen-ppm. Im Reaktionsteil entstandene Mittelsieder, z. B. Dimethoxymethan und/oder Isopren, sind vorzugsweise mit kleiner 200 ppm, bevorzugt kleiner 100 ppm und besonders bevorzugt mit kleiner 50 ppm enthalten. Als weitere Komponenten sind u. a. TBA, Wasser und gegebenenfalls lineare Butene enthalten.

Das Sumpfprodukt **4** der Kolonne (K2) enthält typischer Weise die Hochsieder Diisobuten und MSBE sowie MTBE. Soll in der Kolonne vornehmlich Diisobuten abgetrennt werden, kann der MTBE-Gehalt im Sumpfprodukt auf Werte kleiner 25 Massen-% reduziert werden. Soll zusätzlich MSBE abgetrennt werden, wird bevorzugt aufgrund der kleinen Siedepunktsunterschiede zwischen MSBE und MTBE ein höherer MTBE-Gehalt im Sumpfprodukt, bevorzugt zwischen 60 und 85 Massen-%, zugelassen, um den Aufwand für die Trennung zu reduzieren.

Die Abtrennung von Hochsiedern wie Diisobuten über den Sumpf der Kolonne (K2) hat den Vorteil, dass kein oder nur wenig Hochsieder in den Spaltreaktor gelangen. Dies kann gegebenenfalls den Katalysator vor einer beschleunigten Deakivierung schützen, die sich bei Belegung mit Hochsiedern und gegebenenfalls einer Verkokung ergeben kann.

### Kolonne (K5), Figur 4

Die destillative Trennung erfolgt vorzugsweise unter solchen Bedingungen, dass möglichst reines Methanol als Sumpfprodukt **9** erhalten wird, dass der überwiegende Teil des MTBEs und alle bei der Reaktion entstandenen Mittelsieder wie z. B. Isopren und/der Dimethoxymethan im Kopfprodukt **12** erhalten werden und dass in dem Seitenstrom **11** vorzugsweise mehr als 50 % des im Zulauf **8** enthaltenen Diisobutens, bevorzugt mehr als 80 %, besonders bevorzugt mehr als 95 %, erhalten wird. Das Seitenprodukt stellt gleichzeitig noch einen Auslass für gegebenenfalls im Edukt enthaltenes und bei der Spaltung nicht umgesetztes MSBE dar, sodass eine zu hohe Aufkonzentrierung dieser Komponente im Kreislauf vermieden wird. Durch Begrenzung der Konzentration an MSBE im Kreislauf wird gleichzeitig ein unzulässig hoher Wert an durch Spaltung von MSBE im Reaktionsteil entstehenden linearen Butenen im Produkt Isobuten vermieden. Das Kopfprodukt **12** wird vorzugsweise in die Kolonne (K1) (Figur 4) zurückgefahren.

Wie aus Tabelle 4 zu entnehmen, ist das Isopren-Methanol-Azeotrop der Leichtsieder im System und kann in der eingesetzten Kolonne als Kopfprodukt gewonnen werden. Gleichzeitig können auch Isopren, Dimethoxymethan und die Azeotrope MTBE-Methanol und MSBE-Methanol über Kopf destilliert werden. Diisobuten bildet mit Methanol ein Azeotrop, das zwischen dem MTBE-Methanol-Azeotrop und reinem Methanol siedet. Somit kann das Diisobuten-Methanol-Azeotrop in einen Seitenstrom abgetrennt werden, so dass nahezu Diisobutenfreie Kopf- und Sumpfprodukte in einer Kolonne generiert werden können. Als Sumpfprodukt erhält man so nahezu reines Methanol, das als einzige Nebenkomponente - neben dem in Tabelle 4 nicht aufgeführten Wasser - tert.-Butanol enthält.

Die eingesetzte Destillationskolonne (K5) weist vorzugsweise 20 bis 80 theoretische Trennstufen, bevorzugt 30 bis 70 und besonders bevorzugt 40 bis 60 theoretische Trennstufen auf. Der Seitenstrom kann unterhalb oder oberhalb der Zugabestelle des Kolonnenzulaufs entnommen werden. Bevorzugt erfolgt die Entnahme des Seitenstroms oberhalb, besonders bevorzugt zwischen der 2 und 12 theoretische Stufen oberhalb der Zugabestelle des Kolonnenzulaufs. Vorzugsweise wird die Kolonne, in Abhängigkeit von der realisierten Stufenzahl und von erzielten MTBE-Umsatz, mit einem Rücklaufverhältnis von kleiner 10, bevorzugt von 0,5 bis 5 betrieben. Der Betriebsdruck der Kolonne (K5) wird vorzugsweise auf einen Wert im Bereich von 0,05 bis 1 MPa_{(abs)}, bevorzugt von 0,1 bis 0,3 MPa_{(abs)} eingestellt. Zur Beheizung der Kolonne kann z. B. 0,4 MPa Dampf eingesetzt werden. Die Kondensation kann, je nach gewähltem Betriebsdruck, gegen Kühlsole, Kühlwasser oder Luft erfolgen.

Das erhaltene Sumpfprodukt **9** enthält vorzugsweise über 98 Massen-% Methanol, bevorzugt über 99 Massen-%. Der TBA-Gehalt im Sumpfprodukt beträgt vorzugsweise von 500 bis 3000 Massen-ppm, der Wassergehalt beträgt vorzugsweise von 0,5 bis 0,8 Massen-%. Die im Reaktionsteil entstandenen Mittelsieder, z. B. Isopren und Dimethoxymethan, sind vorzugsweise mit kleiner 200 ppm, bevorzugt kleiner 100 ppm und besonders bevorzugt mit kleiner 50 ppm enthalten. Damit hat das Methanol eine so hohe Reinheit, dass es für übliche technische Synthesen, beispielsweise Veresterungen oder Veretherungen, eingesetzt werden kann. Bei Bedarf kann das Methanol aber auch in einem weiteren Destillationsschritt auf noch höhere Reinheiten aufkonzentriert werden.

Das erhaltene Kopfprodukt **12** enthält vorzugsweise den überwiegenden Teil des im Zulaufstrom **8** enthaltenen MTBE sowie Methanol, die im Reaktionsteil entstandenen Mittelsieder, z. B. Dimethoxymethan und Isopren, und gegebenenfalls MSBE und wird vorzugsweise in die Kolonne (K1) zurückgefahren Der MTBE-Gehalt, liegt, je nach Destillationsbedingungen und Kolonnendruck, vorzugsweise zwischen 65 und 88 Massen-%, bevorzugt zwischen 75 und 85 Massen-%. Der Methanolgehalt liegt vorzugsweise zwischen 10 und 35 %, bevorzugt zwischen 12 und 18 %. Der Gehalt an MSBE liegt vorzugsweise zwischen 0,5 und 5 Massen-%. Enthält der Zulaufstrom **8** Isopren, liegt der Isoprengehalt vorzugsweise kleiner 1 Massen-%. Enthält der Zulaufstrom **8** Dimethoxymethan, liegt der Dimethoxygehalt vorzugsweise kleiner 5000 Massen-ppm.

Der erhaltene Seitenstrom **11** enthält vorzugsweise die nahezu vollständige Menge des im Zulaufstrom **8** enthaltenden Diisobutens. Darüber hinaus kann der Seitenstrom, je nach Fahrweise der Kolonne, zwischen 10 und 40 Massen-% des im Zulaufstrom **8** enthaltenen MSBE enthalten. Da je nach Fahrweise und verwendetem Katalysator das MSBE im Spaltungsreaktor (R) nicht vollständig umgesetzt wird, bestünde bei Rückführung ohne Ausschleusung des Seitenstroms die Gefahr einer unerwünschten Aufkonzentrierung von MSBE im Kreislauf, die durch die Ausschleusung im Seitenstrom sehr einfach vermieden werden kann. Der Seitenstrom **11** kann thermisch verwertet werden, als Einsatzstoff für eine Synthesegasanlage verwendet oder als Kraftstoffkomponente genutzt werden.

### Kolonne (K6), Figuren 1 & 2

In der Kolonne (K6) werden erfindungsgemäß aus dem überwiegend aus MTBE und Methanol bestehenden Rückstrom **10** die im Reaktionsteil entstandenen Mittelsieder, z. B. Dimethoxymethan und/oder Isopren, abgetrennt.

Vorzugsweise wird die Stofftrennung in einer Destillationskolonne durchgeführt, die 20 bis 75 theoretische Trennstufen, bevorzugt 25 bis 55 und besonders bevorzugt 30 bis 45 theoretische Trennstufen aufweist. Vorzugsweise wird die Kolonne, in Abhängigkeit von der realisierten Stufenzahl, der Zusammensetzung des Rückführstroms und der Menge der im Spaltungsreaktor (R) entstandenen Mittelsieder, z. B. Dimethoxymethan und/oder Isopren, mit einem Rücklaufverhältnis zwischen 10 und 150, insbesondere zwischen 30 und 100 betrieben. Die Kolonne wird vorzugsweise mit einem Betriebsdruck von 0,05 bis 1 MPa_{(abs)}, bevorzugt von 0,3 bis 0,6 MPa_{(abs)} betrieben. Zur Beheizung der Kolonne kann z. B. Dampf eingesetzt werden. Die Kondensation kann, je nach gewähltem Betriebsdruck, gegen Kühlsole, Kühlwasser oder Luft erfolgen. Der Kopfbrüden der Kolonne kann vollständig oder nur teilweise kondensiert werden, so dass das Kopfprodukt **14** entweder flüssig oder dampfförmig abgezogen werden kann. Das Kopfprodukt **14** kann thermisch verwertet werden oder als Einsatzstoff einer Synthesegasanlage genutzt werden.

Werden in dem erfindungsgemäßen Verfahren Kolonnen eingesetzt, wie beispielsweise die in den Figuren 1 bis 4 mit K1, K2, K3, K4, K5 und K6 bezeichneten Kolonnen, so können diese mit Einbauten, die z. B. aus Böden, rotierenden Einbauten, regellosen Schüttungen und/oder geordneten Packungen sind, versehen sein.

Bei den Kolonnenböden können z. B. folgende Typen zum Einsatz kommen:
- Böden mit Bohrungen oder Schlitzen in der Bodenplatte.
- Böden mit Hälsen oder Kaminen, die von Glocken, Kappen oder Hauben überdeckt sind.
- Böden mit Bohrungen in der Bodenplatte, die von beweglichen Ventilen überdeckt sind.
- Böden mit Sonderkonstruktionen.

In Kolonnen mit rotierenden Einbauten kann der Rücklauf z. B. durch rotierende Trichter versprüht oder mit Hilfe eines Rotors als Film auf einer beheizten Rohrwand ausgebreitet werden.

Bei Einsatz von Kolonnen mit regellosen Schüttungen mit verschiedenen Füllkörpern können die Füllkörper aus fast allen Werkstoffen, insbesondere aus Stahl, Edelstahl, Kupfer, Kohlenstoff, Steingut, Porzellan, Glas oder Kunststoffen bestehen und die verschiedensten Formen, insbesondere die Form von Kugeln, Ringen mit glatten oder profilierten Oberflächen, Ringen mit Innenstegen oder Wanddurchbrüchen, Drahtnetzringen, Sattelkörper und Spiralen aufweisen.

Packungen mit regelmäßiger/geordneter Geometrie können z. B. aus Blechen oder Geweben bestehen. Beispiele für solche Packungen sind Sulzer Gewebepackungen BX aus Metall oder Kunststoff, Sulzer Lamellenpackungen Mellapak aus Metallblech, Hochleistungspackungen von Sulzer wie Mella-pakPlus, Strukturpackungen von Sulzer (Optiflow), Montz (BSH) und Kühni (Rombopak).

### Isobutenaufarbeitung

Der in Schritt b) des Verfahrens erhaltene isobutenreiche Strom 7 kann zu Isobuten mit höheren Reinheiten aufgearbeitet werden. Dabei werden vor allem Methanol, Dimethylether (DME) und Wasser entfernt. Beispielhaft wird daher eine Aufarbeitung des Stroms **7** aus den in Figuren 1 bis 4 dargestellten bevorzugten Ausführungsformen beschrieben.
Das Methanol im Strom **7** kann nach an sich bekannten Verfahren, beispielsweise durch Extraktion, abgetrennt werden. Die Extraktion von Methanol aus Strom **7** kann beispielsweise mit Wasser oder einer wässrigen Lösung als Extraktionsmittel z. B. in einer Extraktionskolonne durchgeführt werden. Vorzugsweise wird die Extraktion mit Wasser oder einer wässrigen Lösung in einer Extraktionskolonne durchgeführt, die vorzugsweise 4 bis 16 theoretische Trennstufen aufweist. Das Extraktionsmittel durchströmt die Extraktionskolonne in Bezug auf den zu extrahierenden Strom vorzugsweise im Gegenstrom. Die Extraktion wird vorzugsweise bei einer Temperatur von 15 bis 50 °C, bevorzugt 25 bis 40 °C durchgeführt. Beispielweise kann bei der Verwendung einer Extraktionskolonne mit mehr als 6 theoretischen Trennstufen, die bei einem Druck von 0,9 MPa_{(abs)} und einer Temperatur von 40 °C betrieben wird, ein wassergesättigtes Isobuten mit einem Restgehalt an Methanol von unter 500 Massen-ppm, bevorzugt unter 100 Massen-ppm und besonders bevorzugt unter 30 Massen-ppm Methanol erhalten werden.

Der bei der Extraktion erhaltene methanolhaltige Wasserextrakt kann destillativ in Wasser und Methanol getrennt werden. Das Wasser kann als Extraktionsmittel in die Extraktionsstufe zurückgeführt werden. Das Methanol kann für übliche technische Synthesen, beispielsweise Veresterungen oder Veretherungen genutzt werden.

Der feuchte Isobutenstrom aus der Extraktionskolonne kann in einer oder mehreren weiteren Destillationskolonnen durch Abtrennung von Wasser und optional von DME zu trockenem Isobuten aufgearbeitet werden. Das trockene Isobuten wird dabei als Sumpfprodukt erhalten. Im Kondensationssystem am Kopf der Kolonne kann nach einer Phasentrennung Wasser flüssig und DME mit Restmengen Isobuten flüssig und /oder gasförmig abgezogen werden. Eine für die Trocknung bevorzugt eingesetzte Destillationskolonne weist vorzugsweise von 30 bis 80 theoretische Trennstufen, bevorzugt von 40 bis 65 theoretische Trennstufen auf. Das Rücklaufverhältnis beträgt, in Abhängigkeit von der realisierten Stufenzahl und der erforderlichen Reinheit des Isobutens, vorzugsweise kleiner 60, bevorzugt kleiner 40. Der Betriebsdruck der Kolonne K2 kann vorzugsweise zwischen 0,1 und 2,0 MPa_{(abs)} eingestellt werden. Der am Kopf der Kolonne erhaltene DME-reiche Strom kann, falls nötig, weiter destillativ getrennt werden.

Aus dem Strom 7 kann ein Teil des DME optional bereits bei der Destillation (K3) abgetrennt werden, indem der Kondensator an der Destillationskolonne bzw. Reaktivdestillationskolonne als Partialkondensator betrieben wird. In diesem kann die im Kopfprodukt enthaltene C₄-Fraktion kondensiert werden und ein Teil des gasförmigen Dimethylethers als Abgasstrom abgezogen werden.

Eine Aufbereitung von Isobuten durch Extraktion und Destillation ist z. B. in DE 102 38 370 ausführlich beschrieben. Bevorzugt wird aus dem Isobuten aufweisenden Kopfstrom 7 Methanol durch Extraktion und aus dem extrahierten Isobuten DME und gegebenenfalls Wasser destillativ abgetrennt.

Besonders bevorzugt wird im erfindungsgemäßen Verfahren eine Kolonne zur Abtrennung von DME und Wasser mit einem Dekanter zur Abtrennung von Wasser, der sich im Seitenstrom der Kolonne befindet, verwendet. Durch die Einbindung des Dekanters im Seitenstrom der Kolonne können Isobutenverluste minimiert werden. Ein derartiges Verfahren ist beispielsweise auch in der Anmeldung DE 102 38 370 beschrieben. Der feuchte Isobutenstrom aus der Extraktion wird dabei, gegebenenfalls nach Abtrennung von restlichem heterogenem Wasser, beispielsweise durch einen Dekanter oder Koaleszer, in eine Kolonne eingespeist. Am Kopf der Kolonne wird DME und im Sumpf trockenes Isobuten erhalten. Unterhalb oder oberhalb der Edukteinleitungsstelle wird ein Seitenstrom aus der Kolonne flüssig entnommen, der in einen Dekanter geführt wird. Im Dekanter wird die wässrige Phase von der an Wasser verarmten organischen Phase getrennt. Das Wasser wird ausgeschleust, die organische Phase in die Kolonne zurückgeführt. Der Abzug des Stroms zum Seitendekanter erfolgt dabei bevorzugt unterhalb des Kolonnenzulaufs, die Rückführung des Stroms vom Dekanter in die Kolonne unterhalb der Entnahmestelle.
Die Kolonne weist dabei vorzugsweise eine Trennstufenzahl von 30 bis 80 theoretische Trennstufen, bevorzugt von 40 bis 65 theoretische Trennstufen auf. Das zu reinigende Isobuten wird vorzugsweise oberhalb der 15 bis 30 Trennstufe, jeweils gezählt von oben, eingespeist. Bevorzugt wird zwei bis fünf Trennstufen oberhalb der Einspeisestelle das gesamte Kondensat dieser Trennstufe abgezogen und in den Dekanter geleitet. Nach Abtrennung des Wassers wird die organische Phase ein bis zwei Trennstufen tiefer in die Kolonne zurückgeführt. Das Rücklaufverhältnis der Kolonne beträgt, in Abhängigkeit von der realisierten Stufenzahl und der erforderlichen Reinheit des Isobutens, vorzugsweise kleiner 60, bevorzugt kleiner 40. Der Betriebsdruck der Kolonne liegt vorzugsweise zwischen 0,1 und 2,0 MPa_{(abs)}, besonders bevorzugt zwischen 1,0 und 1,5 MPa_{(abs)}.

Das derart gewonnene Isobuten kann z. B. die in Tabelle 5 aufgeführte Zusammensetzung aufweisen:

**Tabelle 5: Typische Zusammensetzung von marktgängigem Isobuten**

| Massenanteile [kg/kg] | |
|---|---|
| C₃-Kohlenwasserstoffe | <0,000100 |
| Butane | <0,001000 |
| Isobuten | >0,999000 |
| 1-Buten / 2-Butene | <0,001000 |
| Methanol | <0,000030 |
| C₅-Kohlenwasserstoffe | <0,000500 |
| Wasser | <0,000050 |
| Oxyenate | <0,000010 |

| | |
|---|---|
| Oxygenate: Beispielsweise DME, Dimethoxymethan | |

Die im Isobuten enthaltenen linearen Butene (1-Buten, 2-Butene) sind von diesem technisch sinnvoll nicht abtrennbar. Die Bildung der linearen Butene erfolgt unter anderem aus der Spaltung von MSBE, welches im MTBE enthalten sein kann. Daher kann durch vollständige Abtrennung von MSBE in der Kolonne (K2) die Bildung der linearen Butene vermieden werden. Um die Destillationskosten zu begrenzen, kann es jedoch vorteilhaft sein, eine geringe Konzentration an MSBE zuzulassen. Dies ist insbesondere dann möglich, wenn in der Schritt a) des Verfahrens ein Katalysator eingesetzt wird, der MTBE schneller als MSBE zersetzt.

Je nach Reinheitsanforderungen sind aber bei Bedarf auch geringere Konzentrationen der Nebenkomponenten erreichbar.

Das mit dem erfindungsgemäßen Verfahren hergestellte Isobuten kann z. B. zur Herstellung von Methacrylsäure, Methylmethacrylat, Diisobuten, Polyisobuten, Alkylphenolen, Methallylchlorid, oder Methallylsulfonaten eingesetzt werden. Insbesondere kann es vorteilhaft sein, sowohl das bei der Spaltung erhaltene Methanol als auch das Isobuten zur Herstellung von Methylmethacrylat einzusetzen. Ein solches Verfahren zur Herstellung von Methylmethacrylat wird beispielsweise in EP 1 254 887 beschrieben, auf welches ausdrücklich verwiesen wird.
Die folgenden Beispiele sollen die Erfindung erläutern.

### Beispiele

### Beispiel 1

Die Spaltung wurde in einem Rohrreaktor mit Heizmantel, durch den ein Wärmeträgeröl (Marlotherm SH der Sasol Olefins & Surfactans GmbH) floss, durchgeführt. Als Katalysator wurde ein mit Magnesium dotiertes Silika-Alumina verwendet. Die Herstellung des Katalysators erfolgte entsprechend der Patentanmeldung DE 10 2006 040432, s. Beispiel 2. Als Edukt wurde MTBE hoher Reinheit eingesetzt, welches normalerweise nicht als Kraftstoffzusatz sondern als Lösungsmittel eingesetzt wird (DRIVERON-S der Firma Evonik Oxeno GmbH).

Vor Eintritt in den Reaktor wurde das MTBE in einem Verdampfer bei 300 °C vollständig verdampft. Die Spaltung wurde bei einer Temperatur von 280 °C (Temperatur des Marlotherms im Zulauf des Reaktormantels) durchgeführt, der Druck wurde über eine Druckhaltung am Ende des Reaktors auf konstant 0,7 MPa_{(abs)} eingestellt. Der MTBE Zulauf wurde auf konstant 1500 g/h eingeregelt, was bei einer Katalysatormenge von 314 g einem WHSV Wert von 4,78 h⁻¹ entspricht. Das den Reaktor verlassende gasförmige Spaltgemisch wurde vollständig kondensiert und gaschromatographisch analysiert.

Nach einer Standzeit von 1006 Stunden betrug der Umsatz des MTBE unter den gewählten Reaktionsbedingungen 98 %. Neben den Spaltprodukten Isobuten und Methanol wurden die in der Tabelle 6 aufgeführten Nebenprodukte nachgewiesen.

**Tabelle 6: Analysen Reaktorzulauf, Reaktoraustrag (jeweils Massenanteile)**

| *Reaktorzulauf* | | | *Reaktoraustrag* | |
|---|---|---|---|---|
| *Komponente* | *Anteil* | | *Spurenkomponenten* | *Anteil* |
| MTBE | 99,949 % | | DME | 0,955 % |
| 2-Methoxybutan | < ng | | C₈-Kohlenwasserstoffe | 0,063 % |
| Isopren | < ng | | Isobutan | 0,010 % |
| C₅-Kohlenwasserstoffe | 0,026 % | | Isopren | 0,003 % |
| Methanol | 0,013 % | | Dimethoxymethan | 0,010 % |
| 3-Methoxy-1-buten | 0,004 % | | 1,3-Butadien | 0,0006 % |
| Rest | 0,008 % | | n-Butene | < 0,0005 % |

| | | | | |
|---|---|---|---|---|
| ng: Nachweisgrenze | | | | |

Es zeigt sich, zusätzlich neben Dimethylether (DME) und C₈-Kohlenwasserstoffen weitere Nebenprodukte (Isopren, Dimethoxymethan, Isobutan) in geringerer Konzentration gebildet werden. Die Bildung von 1,3-Butadien wird auf die Anwesenheit von 3-Methoxy-1-buten im eingesetzten MTBE zurückgeführt. Da der Rohstoff praktisch frei von 2-Methoxybutan (MSBE) ist, werden keine wesentlichen Mengen an linearen Butenen gebildet.

### Erläuterungen zu den Beispielen 2 und 3

In den nachfolgenden Beispielen 2 und 3 wurden Rechnungen mit dem stationären Simulationsprogramm ASPEN Plus (Version 12.1 der Firma AspenTech) durchgeführt, die die Auswirkungen der Mittelsiederbildung im Gesamtprozess wiedergeben.

Um transparente, reproduzierbare Daten zu erzeugen, wurden nur allgemein zugängliche Stoffdaten eingesetzt. Außerdem wurde bei allen Varianten auf den Einsatz einer Reaktivdestillation verzichtet. Durch diese Vereinfachungen ist es dem Fachmann leicht möglich, die Berechnungen nachzuvollziehen. Die eingesetzten Methoden besitzen zwar keine ausreichende Genauigkeit für die Auslegung technischer Anlagen, die qualitativen Unterschiede der Schaltungen werden aber korrekt erfasst. In allen gezeigten Varianten kann der MTBE-Umsatz durch Einsatz einer Reaktivdestillation erhöht werden.

In den Beispielen wurde die Property-Methode "UNIFAC-DMD" (s. J. Gmehling, J. Li, and M. Schiller, Ind. Eng. Chem. Res. 32, (1993), pp. 178-193).benutzt. Für den Reaktor R wird jeweils ein Reaktorvolumen von 100 I modelliert, wobei eine Füllung mit einem Katalysator angenommen wird, der formal aus Magnesiumoxid, Aluminiumoxid und Siliziumoxid besteht und dessen Herstellung in DE 10 2006 040432 beschrieben ist.

Für die Reaktormodellierung wurde in den Rechnungen ein kinetisches Reaktormodell verwendet, das auf umfangreichen experimentellen Messdaten mit diesem Katalysator beruht. In den Beispielen werden daher jeweils auch die Reaktionstemperaturen genannt, die bei der Reaktormodellierung angenommen wurden. Da auch jeweils die Zusammensetzung der ein- und ausgehenden Ströme der Reaktionsstufe genannt werden, ist es dem Fachmann durch Nachstellung der Reaktoren mit fest vorgegebenen Umsätzen möglich, das Beispiel nachzurechnen, ohne die genauen Gleichungen für die Kinetik zu kennen.

### Beispiel 2

Das Beispiel 2 entspricht der in Figur 3 dargestellten Variante. Als Zulauf zu der MTBE-Spaltungsanlage wird gemäß Figur 3 ein MTBE-Strom **1a** (Einsatz-MTBE) von 100 kg/h mit der in Tabelle 7 aufgeführten Zusammensetzung angenommen (typisches Kraftstoff-MTBE, vergleiche mit Tabelle 2).

**Tabelle 7: Zusammensetzung des angenommen MTBE-Eintrittstroms 1a in die MTBE-Spaltungsanlage für Beispiel 2.**

| | Einsatz-MTBE **1a** |
|---|---|
| **Massenstrom [kg/h]** | 100,0 |
| **Massenanteile [kg/kg]** | |
| Dimethylether (DME) | |
| Isobuten | |
| Isobutan | |
| 1-Buten/2-Butene | 0,001000 |
| C₅-Kohlenwasserstoffe | 0,001500 |
| Isopren | |
| Dimethoxymethan | |
| MTBE | 0,979650 |
| 2-Methoxybutan (MSBE) | 0,003000 |
| Methanol | 0,008500 |
| Tert.-Butanol (TBA) | 0,003000 |
| Wasser | 0,000050 |
| Diisobuten | 0,003300 |

Das Einsatz-MTBE **1a** wird mit dem Rückführstrom **10** vermischt und in die Kolonne K1 eingespeist. Bei dem Rückführstrom **10** handelt es sich um den Destillatstrom der Kolonne K4, der die komplette Menge des im Spaltungsreaktor (R) nicht umgesetzten MTBE, die Nebenkomponenten Diisobuten, MSBE und Methanol enthält. Darüber hinaus enthält dieser Strom auch im Spaltungsreaktor entstandene Mittelsieder, in diesem Beispiel Isopren und Dimethoxymethan. Die angenommene Zusammensetzung des Rückstroms **10** und des sich aus der Mischung ergebenen Zulaufstroms zur Kolonne K1 ist in Tabelle 8 dargestellt.

**Tabelle 8: Zusammensetzung des Rückführstroms 10 und des Zulaufstroms der Kolonne K1 für Beispiel 2.**

| | Rückführstrom **10** | Zulauf K1 |
|---|---|---|
| **Massenstrom [kg/h]** | 10,2 | 110,2 |
| **Massenanteile [kg/kg]** | | |
| DME | 0,000256 | 0,000024 |
| Isobuten | 0,002094 | 0,000193 |
| Isobutan | | |
| 1-Buten/2-Butene | 0,000013 | 0,000909 |
| C₅-Kohlenwasserstoffe | | 0,001362 |
| Isopren | 0,002183 | 0,000201 |
| Dimethoxymethan | 0,000384 | 0,000035 |
| MTBE | 0,593166 | 0,944003 |
| MSBE | 0,017945 | 0,004378 |
| Methanol | 0,376157 | 0,042411 |
| Tert.-Butanol | 0,000030 | 0,002726 |
| Wasser | 0,000016 | 0,000047 |
| Diisobuten | 0,007758 | 0,003711 |

Die Aufgabe der Kolonne K1 im Beispiel 2 ist neben der Abtrennung der im Einsatz-MTBE enthaltenen C₄- und C₅-Kohlenwasserstoffe vor allem die Ausschleusung der im Rückführstrom enthaltenen Mittelsieder, die im Spaltungsreaktor (R) entstanden sind, in diesem Beispiel Isopren und Dimethoxymethan. Die Kolonne hat 55 theoretische Stufen und wird bei einem Rücklaufverhältnis von 116 und bei einem Druck von 0,3 MPa_{(abs)} betrieben. Die Stoffzugabe erfolgt oberhalb Stufe 22, von oben gezählt. Die Kopftemperatur beträgt 72,4 °C, die Sumpftemperatur beträgt 87,3 °C. Das Destillat dieser Kolonne 2 hat einen Restgehalt von ca. 68 Massen-% MTBE. Durch Erhöhung des Rücklaufverhältnisses und/oder der Trennstufenzahl ließe sich der MTBE-Gehalt reduzieren. Das Sumpfprodukt ist frei von im MTBE und im Rückführstrom enthaltenen Leichtsiedern (C₄- und C₅-Kohlebwassserstoffen). Ebenfalls abgetrennt werden die Mittelsieder Isopren und Dimethoxymethan.

**Tabelle 9: Zusammensetzung des Destillatstroms 2 und des Sumpfprodukts 3 der Kolonne K1 für Beispiel 2.**

| | Destillat K1 **2** | Sumpfprodukt K1 **3** |
|---|---|---|
| **Massenstrom [kg/h]** | 2,4 | 107,8 |
| **Massenanteile [kg/kg]** | | |
| DME | 0,001097 | |
| Isobuten | 0,008980 | |
| Isobutan | | |
| 1-Buten/2-Butene | 0,042263 | |
| C₅-Kohlenwasserstoffe | 0,063312 | |
| Isopren | 0,009361 | |
| Dimethoxymethan | 0,000822 | 0,000018 |
| MTBE | 0,682321 | 0,949754 |
| MSBE | 0,000073 | 0,004473 |
| Methanol | 0,189593 | 0,039176 |
| TBA | | 0,002786 |
| Wasser | 0,002179 | |
| Diisobuten | | 0,003793 |

Das von Leicht- und Mittelsiedern befreite Sumpfprodukt der Kolonne K1 wird in die Kolonne K2 eingespeist. Die Trennaufgabe der Kolonne K2 ist die Abtrennung von Diisobuten und MSBE. Die Kolonne hat 95 theoretische Stufen und wird bei einem Rücklaufverhältnis von 4,2 und bei einem Druck von 0,95 MPa_{(abs)} betrieben. Die Stoffzugabe erfolgt oberhalb Stufe 32 von oben gezählt. Die Kopftemperatur beträgt 140,5 °C, die Sumpftemperatur beträgt 154,4 °C. Als Kopfprodukt 5 wird eine gasförmige Fraktion erhalten, die frei an Diisobuten ist und nur noch 2100 Massen-ppm MSBE enthält, siehe Tabelle 10. Der Gehalt an MTBE beträgt ca. 95,5 Massen-%. Der Gehalt an MTBE im Sumpfprodukt **4** liegt bei ca. 61,8 Massen-%. Durch Erhöhung des Rücklaufverhältnisses und/oder der Trennleistung ließe sich der Gehalt an MTBE im Sumpfprodukt weiter reduzieren.

**Tabelle 10: Zusammensetzung des Destillatstroms 5 und Sumpfstroms 4 der Kolonne K2 für Beispiel 2.**

| | Destillat K2 **5** | Sumpfprodukt K2 **4** |
|---|---|---|
| **Massenstrom [kg/h]** | 106,0 | 1,8 |
| **Massenanteile [kg/kg]** | | |
| Dimethylether | | |
| Isobuten | | |
| Isobutan | | |
| 1-Buten/2-Butene | | |
| C₅-Kohlenwasserstoffe | | |
| Isopren | | |
| Dimethoxymethan | 0,000018 | |
| MTBE | 0,955228 | 0,618100 |
| MSBE | 0,002100 | 0,148272 |
| Methanol | 0,039822 | |
| Tert.-Butanol | 0,002832 | 0,000012 |
| Wasser | | |
| Diisobuten | | 0,233617 |

Der Destillatstrom **5** der Kolonne K2 wird, nach weiterer Aufheizung auf Reaktionstemperatur, dem Spaltungsreaktor (R) zugeführt. Der Reaktor wird bei 305 °C und 0,85 MPa_{(abs)} gefahren. Bei diesen Reaktionsbedingungen ergibt sich ein MTBE-Umsatz von ca. 94 %, der Umsatz von MSBE beträgt ca. 18 %. Bei diesen hohen Reaktionstemperaturen werden in Nebenreaktionen die Mittelsieder Isopren und Dimethoxymethan gebildet. Bei den Reaktionsbedingungen enthält der Reaktoraustrag ca. 70 ppm Dimethoxymethan und ca. 210 ppm Isopren. Isobutan ist zu ca. 220 pppm enthalten. Die Zusammensetzung des Reaktoraustrags **6** zeigt Tabelle 11.

**Tabelle 11: Zusammensetzung des Reaktoraustrags 6 sowie des Destillatstroms 7 und des Sumpfstroms 8 der Kolonne K3 für Beispiel 2.**

| | Reaktoraustrag **6** | Destillat K3 **7** | Sumpfprodukt K3 **8** |
|---|---|---|---|
| **Massenstrom [kg/h]** | 106,0 | 63,5 | 42,6 |
| **Massenanteile [kg/kg]** | | | |
| DME | 0,004873 | 0,008098 | 0,000061 |
| Isobuten | 0,572525 | 0,955934 | 0,000500 |
| Isobutan | 0,000219 | 0,000366 | |
| 1-Buten/2-Butene | 0,000242 | 0,000402 | 0,000003 |
| C₅-Kohlenwasserstoffe | | | |
| Isopren | 0,000210 | | 0,000521 |
| Dimethoxymethan | 0,000071 | | 0,000176 |
| MTBE | 0,056836 | | 0,141632 |
| MSBE | 0,001720 | | 0,004286 |
| Methanol | 0,359580 | 0,034866 | 0,844037 |
| TBA | 0,000422 | | 0,001051 |
| Wasser | 0,002559 | 0,000333 | 0,005880 |
| Diisobuten | 0,000743 | | 0,001852 |

Der Reaktoraustrag **6** wird teilweise kondensiert und zweiphasig der Kolonne K3 zugeführt. Die Kolonne hat 42 theoretische Stufen und wird bei einem Rücklaufverhältnis von 0,7 und bei einem Druck von 0,65 MPa_{(abs)} betrieben. Die Stoffzugabe erfolgt oberhalb Stufe 28, gezählt von oben. Die Kopftemperatur beträgt 51,3 °C, die Sumpftemperatur beträgt 117,1 C. Das Kopfprodukt 7 ist Isobuten mit einer Reinheit von größer 95 Massen-% Isobuten, siehe Tabelle 11. Isopren wird vollständig über Sumpf abgetrennt, ebenso auch Dimethoxymethan. Die in einer typischen Isobutenspezifikation geforderten Grenzen für lineare Butene (< 1000 Massen-ppm) und C₅-Kohlenwasserstoffe (< 1000 Massen-ppm) werden sicher eingehalten. Durch eine Extraktion mit Wasser kann bei Bedarf das Methanol entfernt werden, das Restwasser und DME können durch eine anschließende Destillation abgetrennt und das Isobuten auf eine Reinheit größer 99,9 Massen-% aufkonzentriert werden.

Das Sumpfprodukt 8 der Kolonne K3 besteht überwiegend aus nicht umgesetztem MTBE (ca. 14 Massen-%) und Methanol (ca. 84 Massen-%). Weiterhin enthält dieser Strom die komplette Menge der durch Reaktion entstandenen Mittelsieder Isopren und Dimethoxymethan. Daneben sind unter anderem nicht umgesetztes MSBE, TBA, Wasser und durch Reaktion entstandenes Diisobuten enthalten. Dieser Strom wird der Kolonne K4 zugeführt.

**Tabelle 12: Zusammensetzung des Sumpfstroms 9 der Kolonne K4 für Beispiel 2.**

| | Sumpfprodukt K4 **9** |
|---|---|
| **Massenstrom [kg/h]** | 32,4 |
| **Massenanteile [kg/kg]** | |
| DME | |
| Isobuten | |
| Isobutan | |
| 1-Buten/2-Butene | |
| C₅-Kohlenwasserstoffe | |
| Isopren | |
| Dimethoxymethan | 0,000111 |
| MTBE | |
| MSBE | |
| Methanol | 0,990796 |
| TBA | 0,001372 |
| Wasser | 0,007719 |
| Diisobuten | |

Die Kolonne K4 hat 35 theoretische Stufen und wird bei einem Rücklaufverhältnis von 1,9 und bei einem Druck von 0,1 MPa_{(abs)} betrieben. Die Stoffzugabe erfolgt oberhalb Stufe 10, gezählt von oben. Die Kopftemperatur beträgt 51,3 °C, die Sumpftemperatur beträgt 64,4 °C. Die Zusammensetzung des Sumpfprodukts **9** zeigt Tabelle 12, die Zusammensetzung des Destillats **10** der Kolonne K4 ist in Tabelle 8 mit aufgeführt. In der Kolonne werden MTBE, MSBE und Diisobuten mit einer gewissen Menge an Methanol über Kopf destilliert. Dabei wird die Azeotropbildung dieser Komponenten mit Methanol ausgenutzt. Zusätzlich werden auch alle Leichtsieder und Mittelsieder (DME, Butene, C₅-Kohlenwasserstoffe, Isopren, Dimethoxymethan) weitestgehend abgetrennt, so dass ein sehr reines Sumpfprodukt mit über 99 Massen-% Methanol gewonnen werden kann. Das Destillat 10 der Kolonne K4 wird dem Zulauf der Kolonne K1 beigemischt.

### Beispiel 3

Das Beispiel 3 entspricht der in Figur 4 dargestellten Variante. Als Zulauf zu der MTBE-Spaltungsanlage wird gemäß Figur 4 ein MTBE-Strom **1a** (Einsatz-MTBE) von 100 kg/h mit der in Tabelle 13 aufgeführten Zusammensetzung angenommen.

Gegenüber Beispiel 2 (vergleiche Tabelle 7) hat dieser Einsatzstrom einen geringeren Gehalt an MSBE und Diisobuten, so dass auf eine Abtrennung dieser Komponenten vor dem Reaktionsteil verzichtet werden kann und eine Ausschleusung gemäß Figur 4 im Seitenstrom der Kolonne K5 erfolgen kann.

**Tabelle 13: Zusammensetzung des MTBE-Eintrittstroms 1a in die MTBE-Spaltungsanlage für Beispiel 3.**

| | Einsatz-MTBE **1a** |
|---|---|
| **Massenstrom [kg/h]** | 100,0 |
| **Massenanteile [kg/kg]** | |
| DME | |
| Isobuten | |
| Isobutan | |
| 1-Buten/2-Butene | 0,001000 |
| C₅-Kohlenwasserstoffe | 0,001500 |
| Isopren | |
| Dimethoxymethan | |
| MTBE | 0,981450 |
| MSBE | 0,002000 |
| Methanol | 0,008500 |
| TBA | 0,003000 |
| Wasser | 0,000050 |
| Diisobuten | 0,002500 |

Das Einsatz-MTBE **1a** wird mit dem Rückführstrom **12** vermischt und in die Kolonne K1 eingespeist. Bei dem Rückführstrom **12** handelt es sich um den Destillatstrom der Kolonne K5, der den Großteil des im Reaktionsteil (R) nicht umgesetzten MTBE, die Nebenkomponenten Diisobuten und MSBE und Methanol enthält. Darüber hinaus enthält dieser Strom auch im Reaktionsteil entstandene Mittelsieder, in diesem Beispiel Isopren und Dimethoxymethan. Die Zusammensetzung des Rückstroms **12** und des sich aus der Mischung ergebenen Zulaufstroms zur Kolonne K1 ist in Tabelle 14 dargestellt.

**Tabelle 14: Zusammensetzung des Rückführstroms 12 und des Zulaufstroms der Kolonne K1 für Beispiel 3.**

| | Rückführstrom **12** | Zulauf K1 |
|---|---|---|
| **Massenstrom [kg/h]** | 6,2 | 106,2 |
| **Massenanteile [kg/kg]** | | |
| DME | 0,000291 | 0,000017 |
| Isobuten | 0,003306 | 0,000193 |
| Isobutan | | |
| 1-Buten/2-Butene | 0,000021 | 0,000943 |
| C₅-Kohlenwasserstoffe | 0,000085 | 0,001417 |
| Isopren | 0,003550 | 0,000207 |
| Dimethoxymethan | 0,000620 | 0,000036 |
| MTBE | 0,824913 | 0,972322 |
| MSBE | 0,005645 | 0,002213 |
| Methanol | 0,161560 | 0,017425 |
| TBA | | 0,002825 |
| Wasser | 0,000009 | 0,000048 |
| Diisobuten | | 0,002354 |

Die Aufgabe der Kolonne K1 ist neben der Abtrennung der im Einsatz-MTBE enthaltenen C₄- und C₅-Kohlenwasserstoffe vor allem die Ausschleusung der im Rückführstrom enthaltenen Mittelsieder, die im Reaktionsteil entstanden sind, in diesem Beispiel Isopren und Dimethoxymethan. Die Kolonne hat 60 theoretische Stufen und wird bei einem Rücklaufverhältnis von 197 und bei einem Druck von 0,3 MPa_{(abs)} betrieben. Die Stoffzugabe erfolgt oberhalb Stufe 25, von oben gezählt. Die Kopftemperatur beträgt 48,2 °C, die Sumpftemperatur beträgt 105,8 °C. Das Destillat 2 dieser Kolonne hat einen Restgehalt von 15 Massen-% MTBE. Durch Erhöhung des Rücklaufverhältnisses und/oder der Trennstufenzahl ließe sich der MTBE-Gehalt weiter reduzieren. Das Sumpfprodukt ist frei von im MTBE und im Rückführstrom enthaltenen Leichtsiedern (C₄- und C₅-Kohlenwassserstoffen). Ebenfalls vollständig abgetrennt werden die Mittelsieder Isopren und Dimethoxymethan.

**Tabelle 15: Zusammensetzung des Destillatstroms 2 und des Sumpfprodukts 3 der Kolonne K1 für Beispiel 3.**

| | Destillat K1 **2** | Sumpfprodukt K1 **3** |
|---|---|---|
| **Massenstrom [kg/h]** | 0,4 | 105,8 |
| **Massenanteile [kg/kg]** | | |
| DME | 0,004469 | |
| Isobuten | 0,050785 | |
| Isobutan | | |
| 1-Buten/2-Butene | 0,248372 | |
| C₅-Kohlenwasserstoffe | 0,372063 | |
| Isopren | 0,054157 | |
| Dimethoxymethan | 0,008717 | |
| MTBE | 0,150000 | 0,975456 |
| MSBE | 0,000017 | 0,002221 |
| Methanol | 0,111419 | 0,017067 |
| TBA | | 0,002836 |
| Wasser | | 0,000048 |
| Diisobuten | | 0,002363 |

Das von Leicht- und Mittelsiedern befreite Sumpfprodukt der Kolonne K1 wird flüssig auf Reaktionsdruck verdichtet, aufgeheizt und verdampft und gasförmig, mit einer Temperatur, die der Reaktionstemperatur im Reaktor entspricht, dem Reaktionsteil (R) zugeführt. Reaktionstemperatur, Reaktordruck, Umsätze und Nebenproduktspektrum bleiben gegenüber Beispiel 2 unverändert. Damit enthält der der Reaktoraustrag ca. 60 ppm Dimethoxymethan und ca. 210 ppm Isopren. Isobutan ist zu ca. 220 pppm enthalten. Die Zusammensetzung des Reaktoraustrags **6** zeigt Tabelle 16.

**Tabelle 16: Zusammensetzung des Reaktoraustrags 6 sowie des Destillatstroms 7 und des Sumpfstroms 8 der Kolonne K3 für Beispiel 3.**

| | Reaktoraustrag **6** | Destillat K3 **7** | Sumpfprodukt K3 **8** |
|---|---|---|---|
| **Massenstrom [kg/h]** | 105,8 | 64,7 | 41,1 |
| **Massenanteile [kg/kg]** | | | |
| DME | 0,004662 | 0,007599 | 0,000044 |
| Isobuten | 0,584796 | 0,956378 | 0,000500 |
| Isobutan | 0,000219 | 0,000359 | |
| 1-Buten/2-Butene | 0,000256 | 0,000417 | 0,000003 |
| C₅-Kohlenwasserstoffe | 0,000005 | | 0,000013 |
| Isopren | 0,000212 | | 0,000543 |
| Dimethoxymethan | 0,000056 | | 0,000143 |
| MTBE | 0,058040 | | 0,149304 |
| MSBE | 0,001819 | | 0,004680 |
| Methanol | 0,344041 | 0,034895 | 0,830158 |
| TBA | 0,000423 | | 0,001087 |
| Wasser | 0,002525 | 0,000351 | 0,005944 |
| Diisobuten | 0,002947 | | 0,007581 |

Der Reaktoraustrag **6** wird teilweise kondensiert und zweiphasig der Kolonne K3 zugeführt. Stufenzahl, Stoffzugabestelle, Rücklaufverhältnis und Betriebsdruck der Kolonne K3 bleiben gegenüber Beispiel 2 unverändert. Die Kopftemperatur beträgt 51,3 °C, die Sumpftemperatur beträgt 116,9 C. Das Kopfprodukt 7 ist Isobuten mit einer Reinheit von größer 95 Massen-% Isobuten, siehe Tabelle 16. Isopren wird vollständig über Sumpf abgetrennt, ebenso auch Dimethoxymethan. Die in einer typischen Isobutenspezifikation geforderten Grenzen für lineare Butene (< 1000 Massen-ppm) und C₅-Kohlenwasserstoffe (< 1000 Massen-ppm) werden sicher eingehalten. Durch eine Extraktion mit Wasser kann bei Bedarf das Methanol entfernt werden, das Restwasser und DME können durch eine anschließende Destillation abgetrennt und das Isobuten auf eine Reinheit größer 99,9 Massen-% aufkonzentriert werden.

Das Sumpfprodukt **8** der Kolonne K3 besteht überwiegend aus nicht umgesetztem MTBE (ca. 15 Massen-%) und Methanol (ca. 84 Massen-%). Weiterhin enthält dieser Strom die komplette Menge der durch die Reaktion entstandenen Mittelsieder Isopren und Dimethoxymethan. Daneben sind unter anderem nicht umgesetztes MSBE, TBA, Wasser und durch Reaktion entstandenes Diisobuten enthalten. Dieser Strom wird der Kolonne K5 zugeführt.

**Tabelle 17: Zusammensetzung des Sumpfstroms 9 und des Seitenstroms 11 der Kolonne K5 für Beispiel 3.**

| | Sumpfprodukt K5 **9** | Seitenstrom K5 **11** |
|---|---|---|
| **Massenstrom [kg/h]** | 30,4 | 4,5 |
| **Massenanteile [kg/kg]** | | |
| DME | | |
| Isobuten | | |
| Isobutan | | |
| 1-Buten/2-Butene | | |
| C₅-Kohlenwasserstoffe | | |
| Isopren | | 0,000076 |
| Dimethoxymethan | | 0,000186 |
| MTBE | | 0,229335 |
| MSBE | | 0,034997 |
| Methanol | 0,990493 | 0,666034 |
| TBA | 0,001457 | 0,000077 |
| Wasser | 0,008030 | 0,000008 |
| Diisobuten | | 0,069283 |

Die Kolonne K5 hat 62 theoretische Stufen und wird bei einem Rücklaufverhältnis von 14,5 und bei einem Druck von 0,15 MPa_{(abs)} betrieben. Die Stoffzugabe erfolgt oberhalb Stufe 36, gezählt von oben. Der Seitenstrom wird flüssig auf Stufe 25, gezählt von oben, entnommen. Die Kopftemperatur beträgt 61,0 °C, die Sumpftemperatur beträgt 75,1 °C. Die Zusammensetzung des Sumpfprodukts **9** und des Seitenstroms **11** zeigt in Tabelle 17, die Zusammensetzung des Destillats **10** der Kolonne K4 ist in Tabelle 14 mit aufgeführt.

In der Kolonne wird der überwiegende Teil des im Zulaufstrom **8** enthaltenen MTBE sowie MSBE mit einer geringen Menge an Methanol über Kopf destilliert. Dabei wird die Azeotropbildung dieser Komponenten mit Methanol ausgenutzt. Zusätzlich werden auch alle Leichtsieder (DME, Butene und Pentane) und die im Reaktionsteil entstandenen Mittelsieder (Isopren, Dimethoxymethan) über Kopf abgetrennt. Das Destillat **12** wird in den Reaktionsteil zurückgeführt.

Der Seitenstrom **11** enthält das komplette Diisobuten, zusätzlich MTBE, Methanol und MSBE sowie die Mittelsieder Isopren und Dimethoxymethan in Spuren. Durch die Ausschleusung von MSBE kann sich diese Komponente nicht im Kreislauf auf unerwünschte Konzentrationen anreichern. Durch Begrenzung der Konzentration an MSBE im Kreislauf wird damit gleichzeitig ein unzulässig hoher Wert an durch Spaltung von MSBE im Reaktionsteil entstehenden linearen Butenen im Isobutenprodukt vermieden.

Als Sumpfprodukt **9** wird ein sehr reines Methanol gewonnen, das eine Reinheit von über 99 Massen-% aufweist und frei von Diisobuten ist. Als einzige Nebenkomponenten sind TBA und Wasser enthalten. Bei Bedarf kann das Methanol in einem weiteren Destillationsschritt von TBA und Wasser befreit werden und so auf noch höhere Reinheiten aufkonzentriert werden.

## Patentansprüche

1. Verfahren zur Herstellung von Isobuten, aus MTBE-haltigen Gemischen, das folgende Schritte umfasst:
a) Spaltung in einem Reaktor R eines aus einem MTBE-haltigen Einsatzstoff und/oder einem MTBE-haltigen Strom erhaltenen Gemisches, liefernd einen Strom 6 von Reaktionsprodukten, bestehend aus Isobuten, Methanol, MTBE sowie Nebenprodukten, wobei diese aus
a1) Hochsiedern, mit einem Siedebereich oberhalb von 55 °C bei einem Druck von 0,1 MPa;
a2) Mittelsiedern, mit einem Siedebereich von 12 bis 55 °C bei einem Druck von 0,1 MPa; und
a3) Leichtsiedern, mit einem Siedebereich unterhalb von 12 °C bei einem Druck von 0,1 MPa bestehen;
b) destillative Auftrennung des Stroms 6 in einen Strom 7, der das Produkt Isobuten und Leichtsieder enthält, und einen Strom 8, der MTBE, Methanol, Mittelsieder und Hochsieder enthält;
c) destillative Auftrennung des Stroms 8 unter Erhalt eines MTBE-haltigen Stroms 10, 12 und eines methanolhaltigen Hochsieder-Stroms;
d) Rückführung eines MTBE-haltigen Stroms in Schritt a) des Verfahrens, wobei die vollständige oder teilweise Abtrennung der Mittelsieder vor Schritt d) erfolgt aus den mittelsiederreichen Strömen 10,12.

2. Verfahren nach Anspruch 1, **dadurch gekennzeichnet, dass** die Spaltung an einem festen Katalysator erfolgt.

3. Verfahren nach den Ansprüchen 1 bis 2, **dadurch gekennzeichnet, dass** die Spaltung des MTBE-haltigen Gemisches im Temperaturbereich von 150 bis 500 °C durchgeführt wird.

4. Verfahren nach den Ansprüchen 1 bis 3, **dadurch gekennzeichnet, dass** die Spaltung des MTBE-haltigen Gemisches in einem Druckbereich von 0,05 bis 2 MPa durchgeführt wird.

5. Verfahren nach den Ansprüchen 1 bis 4, **dadurch gekennzeichnet, dass** die Spaltung bei einer Eintrittstemperatur in den Reaktor von mindestens 150°C erfolgt.

6. Verfahren nach den Ansprüchen 1 bis 5, **dadurch gekennzeichnet, dass** der der Spaltung zugeführte Strom an MTBE-haltigem Einsatzstoff zuvor einer Destillation unterworfen wird dergestalt, dass der Betriebsdruck dieser Destillation mindestens 0,05 MPa über dem Betriebsdruck der Spaltungsreaktion liegt.

7. Verfahren nach den Ansprüchen 1 bis 6, **dadurch gekennzeichnet, dass** die der Spaltung nachgeschaltete Destillation des Stroms 6 bei einem Betriebsdruck von mindestens 0,05 MPa unter dem Betriebsdruck der Spaltungsreaktion durchgeführt wird.

8. Verfahren nach den Ansprüchen 1 bis 7, **dadurch gekennzeichnet, dass** der Strom 10 mit dem MTBE-haltigen Einsatzstoff zusammengeführt wird und aus diesem Gesamtstrom Hochsieder, Mittelsieder und/oder Leichtsieder abgetrennt werden.

9. Verfahren nach den Ansprüchen 1 bis 8, **dadurch gekennzeichnet, dass** in der ersten Destillationskolonne die Leichtsieder und Mittelsieder als Kopfprodukt und in einer weiteren Destillationskolonne die Hochsieder als Sumpfprodukt abgetrennt werden.

10. Verfahren nach den Ansprüchen 1 bis 9, **dadurch gekennzeichnet, dass** die Leichtsieder Dimethylether und/oder C₄-Kohlenwasserstoffe enthalten.

11. Verfahren nach den Ansprüchen 1 bis 10, **dadurch gekennzeichnet, dass** die Mittelsieder Dimethoxymethan und/oder C₅-Kohlenwasserstoffe enthalten.

12. Verfahren nach den Ansprüchen 1 bis 11, **dadurch gekennzeichnet, dass** die Mittelsieder Isopren enthalten.

13. Verfahren nach den Ansprüchen 1 bis 12 **dadurch gekennzeichnet, dass** die Hochsieder 2-Methoxybutan und/oder C₈-Kohienwasserstoffe enthalten.

## Claims

1. Process for preparing isobutene from MTBE-containing mixtures, which comprises the following steps:
a) in a reactor R, cleaving a mixture obtained from an MTBE-containing feedstock and/or an MTBE-containing stream, affording a stream 6 of reaction products consisting of isobutene, methanol, MTBE and by-products, the latter consisting of
a1) high boilers having a boiling range above 55°C at a pressure of 0.1 MPa;
a2) medium boilers having a boiling range of 12 to 55°C at a pressure of 0.1 MPa; and
a3) low boilers having a boiling range below 12°C at a pressure of 0.1 MPa;
b) distillatively separating stream **6** into a stream **7** which contains the isobutene product and low boilers, and a stream **8** which contains MTBE, methanol, medium boilers and high boilers;
c) distillatively separating stream **8** to obtain an MTBE-containing stream **10, 12** and a methanol-containing high boiler stream;
d) recycling an MTBE-containing stream into step a) of the process, the medium boilers being removed completely or partially before step d) from the streams **10, 12** rich in medium boilers.

2. Process according to Claim 1, **characterized in that** the cleavage is effected over a solid catalyst.

3. Process according to either of Claims 1 and 2, **characterized in that** the cleavage of the MTBE-containing mixture is carried out within the temperature range from 150 to 500°C.

4. Process according to any of Claims 1 to 3, **characterized in that** the cleavage of the MTBE-containing mixture is carried out within a pressure range from 0.05 to 2 MPa.

5. Process according to any of Claims 1 to 4, **characterized in that** the cleavage is effected at an entrance temperature into the reactor of at least 150°C.

6. Process according to any of Claims 1 to 5, **characterized in that** the stream of MTBE-containing feedstock fed to the cleavage is subjected beforehand to a distillation in such a way that the operating pressure of this distillation is at least 0.05 MPa above the operating pressure of the cleavage reaction.

7. Process according to any of Claims 1 to 6, **characterized in that** the distillation of stream 6 which is connected downstream of the cleavage, is carried out at an operating pressure of at least 0.05 MPa below the operating pressure of the cleavage reaction.

8. Process according to any of Claims 1 to 7, **characterized in that** the stream 10 is combined with the MTBE-containing feedstock and high boilers, medium boilers and/or low boilers are removed from this overall stream.

9. Process according to any of Claims 1 to 8, **characterized in that** the low boilers and medium boilers are removed as the top product in the first distillation column, and the high boilers as the bottom product in a further distillation column.

10. Process according to any of Claims 1 to 9, **characterized in that** the low boilers comprise dimethyl ether and/or C₄ hydrocarbons.

11. Process according to any of Claims 1 to 10, **characterized in that** the medium boilers comprise dimethoxymethane and/or C₅ hydrocarbons.

12. Process according to any of Claims 1 to 11, **characterized in that** the medium boilers comprise isoprene.

13. Process according to any of Claims 1 to 12, **characterized in that** the high boilers comprise 2-methoxybutane and/or C₈ hydrocarbons.

## Revendications

1. Procédé pour la préparation d'isobutène à partir de mélanges contenant du MTBE, comprenant les étapes suivantes
a) dissociation dans un réacteur R d'un mélange obtenu à partir d'une substance de départ contenant du MTBE et/ou d'un flux contenant du MTBE, fournissant un flux 6 de produits de réaction, constitué d'isobutène, de méthanol, de MTBE ainsi que de produits secondaires, ceux-ci étant constitués par
a1) des substances à point d'ébullition élevé, présentant une plage d'ébullition supérieure à 55°C à une pression de 0,1 MPa ;
a2) des substances à point d'ébullition moyen, présentant une plage d'ébullition de 12 à 55°C à une pression de 0,1 MPa ; et
a3) des substances à bas point d'ébullition, présentant une plage d'ébullition inférieure à 12°C à une pression de 0,1 MPa ;
b) séparation par distillation du flux 6 en un flux 7, qui contient le produit isobutène et les substances à bas point d'ébullition, et en flux 8 qui contient le MTBE, le méthanol, les substances à point d'ébullition moyen et les substances à point d'ébullition élevé ;
c) séparation par distillation du flux 8 avec obtention d'un flux 10, 12 contenant le MTBE et d'un flux de substances à point d'ébullition élevé contenant le méthanol ;
d) recyclage d'un flux contenant du MTBE dans l'étape a) du procédé,
la séparation complète ou partielle des substances à point d'ébullition moyen ayant lieu avant l'étape d) à partir des flux riches en substances à point d'ébullition moyen 10, 12.

2. Procédé selon la revendication 1, **caractérisé en ce que** la dissociation a lieu sur un catalyseur solide.

3. Procédé selon les revendications 1 à 2, **caractérisé en ce que** la dissociation du mélange contenant du MTBE est réalisée dans une plage de température de 150 à 500°C.

4. Procédé selon les revendications 1 à 3, **caractérisé en ce que** la dissociation du mélange contenant du MTBE est réalisée dans une plage de pression de 0,05 à 2 MPa.

5. Procédé selon les revendications 1 à 4, **caractérisé en ce que** la dissociation a lieu à une température d'entrée dans le réacteur d'au moins 150°C.

6. Procédé selon les revendications 1 à 5, **caractérisé en ce que** le flux alimenté dans la dissociation de substance de départ contenant du MTBE est soumis au préalable à une distillation de manière telle que la pression d'exploitation de cette distillation est supérieure d'au moins 0,05 MPa à la pression d'exploitation de la réaction de dissociation.

7. Procédé selon les revendications 1 à 6, **caractérisé en ce que** la distillation du flux 6 disposée en aval de la dissociation est réalisée à une pression d'exploitation inférieure d'au moins 0,05 MPa à la pression d'exploitation de la réaction de dissociation.

8. Procédé selon les revendications 1 à 7, **caractérisé en ce que** le flux 10 est rassemblé avec la substance de départ contenant du MTBE ; et les substances à point d'ébullition élevé, à point d'ébullition moyen et à bas point d'ébullition sont séparées de ce flux total.

9. Procédé selon les revendications 1 à 8, **caractérisé en ce que** les substances à bas point d'ébullition et à point d'ébullition moyen sont séparées dans la première colonne de distillation en tant que produit de tête et les substances à point d'ébullition élevé sont séparées dans une autre colonne de distillation sous forme de produit de fond.

10. Procédé selon les revendications 1 à 9, **caractérisé en ce que** les substances à bas point d'ébullition contiennent du diméthyléther et/ou des hydrocarbures en C₄.

11. Procédé selon les revendications 1 à 10, **caractérisé en ce que** les substances à point d'ébullition moyen contiennent du diméthoxyméthane et/ou des hydrocarbures en C₅.

12. Procédé selon les revendications 1 à 11, **caractérisé en ce que** les substances à point d'ébullition moyen contiennent de l'isoprène.

13. Procédé selon les revendications 1 à 12, **caractérisé en ce que** les substances à point d'ébullition élevé contiennent du 2-méthoxybutane et/ou des hydrocarbures en C₈.
